# EUROPEAN PATENT APPLICATION

(11) **EP 4 600 355 A1**
(43) Date of publication of application: **13.08.2025**
(21) Application number: 23874687.9
(22) Date of filing: 25.09.2023
(51) Int. Cl.: C12N 15/10, C12Q 1/686

(54) **NUCLEIC ACID EXTRACTION METHOD, NUCLEIC ACID AMPLIFICATION METHOD, NUCLEIC ACID EXTRACTION KIT, AND PCR TEST KIT**

(30) Priority: 06.10.2022 JP 2022161370
(71) Applicant: National Institute for Materials Science, Tsukuba-shi, Ibaraki 305-0047 (JP); Egyptian Liver Research Institute and Hospital, Mansoura, AL-Dakahlia 35511 (EG)
(72) Inventor: EBARA Mitsuhiro, Tsukuba-shi, Ibaraki 305-0047 (JP); TOLBA Ahmed Nabil Ahmed Elsayed, Tsukuba-shi, Ibaraki 305-0047 (JP); Gamal Elsayed Shiha, Mansoura, AL- Dakahlia, 35511 (EG); Ayman Abdel-Fattah Abdel-baky Hassan, Mansoura, AL- Dakahlia, 35511 (EG)
(74) Representative: J A Kemp LLP
(86) International application number: PCT/JP2023/034639
(87) International publication number: WO 2024/075559

(57) **Abstract**

A nucleic acid extraction method includes, in the absence of a proteolytic enzyme: preparing a mixture solution containing at least one specimen selected from the group consisting of cells, extracellular vesicles, and virions, a copolymer including a repeating unit represented by formula 1 described later and a repeating unit represented by formula 2 described later, and an antibody-linker complex obtained by binding an antibody that binds to the specimen and a linker represented by formula 3 described later via an amide bond, and generating an antibody-copolymer conjugate; and heating the mixture solution to allow the antibody-copolymer conjugate to aggregate, and extracting a nucleic acid encapsulated in the specimen. The nucleic acid extraction method can easily extract a nucleic acid encapsulated in a specimen even in the absence of a proteolytic enzyme and even without performing concentration with a carrier nucleic acid.

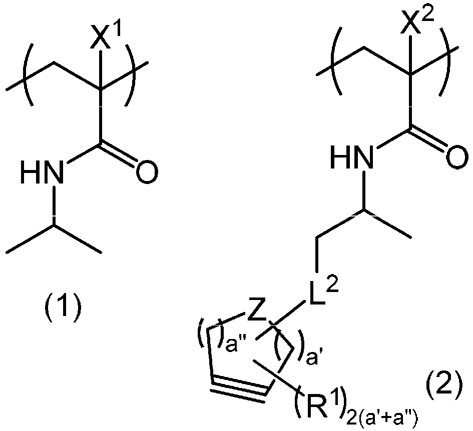

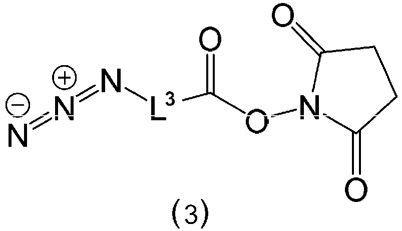

## Description

### Field

The present disclosure relates to a nucleic acid extraction method, a nucleic acid amplification method, a nucleic acid extraction kit, and a PCR test kit.

### Background

Biological diagnostic, testing, and analytical operations require detection of nucleic acids in cells and/or virus particles included in biological samples **(e.g.,** swabs). Generally, in such tests, separation, concentration, and purification of a nucleic acid from an object (specimen such as virus) included in the sample are performed as pretreatment.

Nucleic acids in a sample may form a complex assembly with impurities and the like, and their concentration and purification often require digestion of the impurities with proteolytic enzymes and increase in yield (hereinafter also referred to as "concentration") with "carrier nucleic acids" such as polyadenylic acid. This tendency is particularly evident when the specimen concentration is low. As used herein "specimen" refers to a cell and/or a virus particle or the like that has a nucleic acid to be extracted and is included in a biological sample.

For example, in a polymerase chain reaction (PCR) method test (hereinafter also referred to as "PCR test") performed for isolation and identification for SARS-CoV-2 infection, treatment with a proteolytic enzyme and/or concentration of a specimen and/or a nucleic acid with a carrier nucleic acid were sometimes performed as pretreatment for reverse transcription and amplification of virus ribonucleic acid (RNA) (for example, PTL 1).

### Citation List

### Patent Literature

PTL 1: Japanese Unexamined Patent Application Publication No. 2004-215676

### Summary

### Technical Problem

The treatment of a sample with a proteolytic enzyme and the concentration with addition of a carrier nucleic acid (hereinafter also referred to as "treatment with a proteolytic enzyme and the like") require expensive reagents, complicated experimental manipulations, and a prescribed amount of time. The treatment with a proteolytic enzyme and the like therefore can be a bottleneck in PCR tests. In addition, inventory management of various pretreatment reagents used for the treatment with a proteolytic enzyme and the like is complicated, and the reagents are expensive, leading to increased testing costs.

An object of the present disclosure is to provide a nucleic acid extraction method that can easily extract a nucleic acid encapsulated in a specimen even in the absence of a proteolytic enzyme and even without performing concentration with a carrier nucleic acid. In other words, a nucleic acid extraction method that enables purification and concentration of a specimen for nucleic acid extraction in a single step is provided. Other objects of the present disclosure are to provide a nucleic acid amplification method, a nucleic acid extraction kit, and a PCR test kit.

### Solution to Problem

One embodiment of a nucleic acid extraction method of the present disclosure includes, in the absence of a proteolytic enzyme: preparing a mixture solution including at least one specimen selected from the group consisting of cells, extracellular vesicles, and virions, a copolymer including a repeating unit represented by formula 1 below and a repeating unit represented by formula 2 below, and an antibody-linker complex obtained by binding an antibody that binds to the specimen and a linker represented by formula 3 below via an amide bond, and generating an antibody-copolymer conjugate; and heating the mixture solution to allow the antibody-copolymer conjugate to aggregate, and extracting a nucleic acid encapsulated in the specimen.

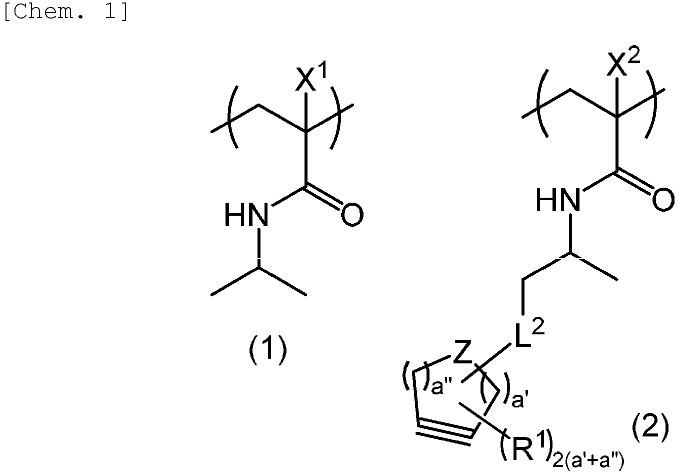

In formula 1, X¹ represents a hydrogen atom or a linear or branched alkyl group having 1 to 6 carbon atoms; in formula 2, X² represents a hydrogen atom or a linear or branched alkyl group having 1 to 6 carbon atoms, L² represents a divalent group, R¹ is selected from the group consisting of a hydrogen atom, a halogen atom, -OR⁵, -NO₂, -CN, -S(O)₂R⁵, an alkyl group having 1 to 24 carbon atoms, an alkenyl group having 2 to 24 carbon atoms, and a (hetero)aryl group having 6 to 24 carbon atoms, a plurality of R¹s are the same or different from each other, two or more of R₁s are optionally bonded to each other to form a ring, where R⁵ is selected from the group consisting of a hydrogen atom, a halogen atom, an alkyl group having 1 to 24 carbon atoms, and a (hetero)aryl group having 6 to 24 carbon atoms, Z is selected from the group consisting of C(R¹)₂, O, S, and NR¹, a' is an integer of 0 to 8, a" is an integer of 0 to 8, and a sum of a' and a" is less than 10.

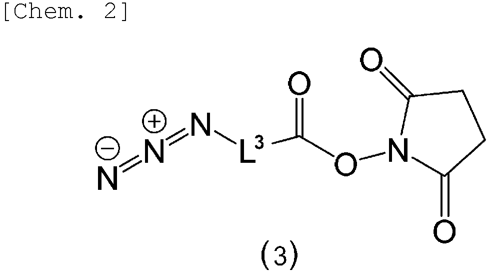

In formula 3, L³ represents a divalent hydrocarbon group optionally having a heteroatom.

One embodiment of a nucleic acid extraction kit of the present disclosure is a nucleic acid extraction kit to be used for extracting a nucleic acid from at least one specimen selected from the group consisting of cells, extracellular vesicles, and virions, in the absence of a proteolytic enzyme. The nucleic acid extraction kit includes: a first agent including a copolymer including a repeating unit represented by formula 1 below and a repeating unit represented by formula 2 below; and a second agent including an antibody-linker complex obtained by binding an antibody that binds to the specimen and a linker represented by formula (3) below via an amide bond.

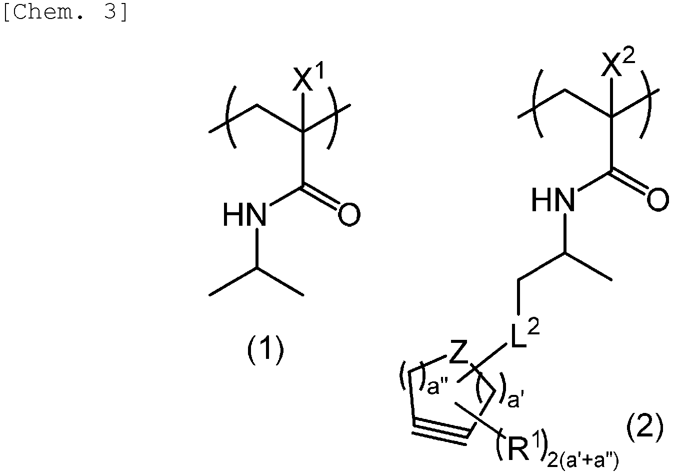

In formula 1, X¹ represents a hydrogen atom or a linear or branched alkyl group having 1 to 6 carbon atoms; in formula 2, X² represents a hydrogen atom or a linear or branched alkyl group having 1 to 6 carbon atoms, L² represents a divalent group, R¹ is selected from the group consisting of a hydrogen atom, a halogen atom, -OR⁵, -NO₂, -CN, -S(O)₂R⁵, an alkyl group having 1 to 24 carbon atoms, an alkenyl group having 2 to 24 carbon atoms, and a (hetero)aryl group having 6 to 24 carbon atoms, a plurality of R¹s are the same or different from each other, two or more of R¹s are optionally bonded to each other to form a ring, where R⁵ is selected from the group consisting of a hydrogen atom, a halogen atom, an alkyl group having 1 to 24 carbon atoms, and a (hetero)aryl group having 6 to 24 carbon atoms, Z is selected from the group consisting of C(R¹)₂, O, S, and NR¹, a' is an integer of 0 to 8, a" is an integer of 0 to 8, and a sum of a' and a" is less than 10.

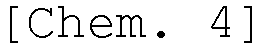

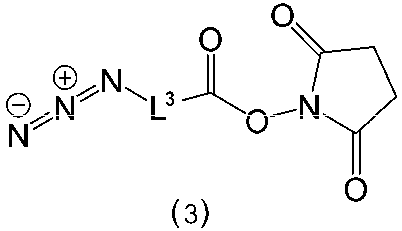

In formula 3, L³ represents a divalent hydrocarbon group optionally having a heteroatom.

### Advantageous Effects of Invention

The present disclosure provides a nucleic acid extraction method that can easily extract a nucleic acid encapsulated in a specimen even in the absence of a proteolytic enzyme and even without performing concentration with a carrier nucleic acid. In other words, the present disclosure provides a nucleic acid extraction method that enables purification and concentration of a specimen for nucleic acid extraction in a single step. The present disclosure also provides a nucleic acid amplification method, a nucleic acid extraction kit, and a PCR test kit.

### Brief Description of Drawings

[Fig. 1] Fig. 1 is a flowchart illustrating a procedure of a nucleic acid extraction method according to the present invention.
[Fig. 2] Fig. 2 illustrates a quantification result by a real-time PCR method using nucleic acids extracted by a method of Comparative Example 1 and a method of Reference Example 1.
[Fig. 3] Fig. 3 illustrates a quantification result by a real-time PCR method using nucleic acids extracted by a method of Comparative Example 1 and a method of Example 1.

### Description of Embodiments

A nucleic acid extraction method according to a first embodiment of the present disclosure includes, in the absence of a proteolytic enzyme: preparing a mixture solution including at least one specimen selected from the group consisting of cells, extracellular vesicles, and virions, a copolymer including a repeating unit represented by formula 1 described later and a repeating unit represented by formula 2 described later, and an antibody-linker complex obtained by binding an antibody that binds to the specimen and a linker represented by formula 3 described later via an amide bond, and generating an antibody-copolymer conjugate; and heating the mixture solution to allow the antibody-copolymer conjugate to aggregate, and extracting a nucleic acid encapsulated in the specimen.

The antibody-copolymer conjugate has water solubility and a lower critical solution temperature (LCST) due to the structure of the copolymer. Thus, the solubility in water decreases upon heating, thereby facilitating precipitation and aggregation.

A specimen (antigen) is specifically recognized by and bound to an antibody through an antigen-antibody interaction, and when heated in this state, the specimen aggregates and is concentrated while bound to the conjugate.

With the nucleic acid extraction method according to the first embodiment, the target nucleic acid can be easily extracted even from a sample with a low antigen concentration and/or without performing pretreatment using a proteolytic enzyme.

According to a second embodiment of the nucleic acid extraction method of the present disclosure, in the nucleic acid extraction method according to the first embodiment, the content of the repeating unit represented by formula 2 described later is 1.0 to 30.0 mol% when the total of repeating units of the copolymer is defined as 100 mol%.

The repeating unit represented by formula 2 (unit 2) has two functions in the copolymer (and the antibody-copolymer conjugate). One of the functions is a function as a binding site with an antibody, and the other is an LCST adjusting function. For the former, the unit 2 has one site (click reaction site) including cyclic alkyne (alkynylene group). Thus, it can easily bind to the azido of the linker of formula 3 through a click reaction. As described later, the linker of formula 3 can bind to an antibody. The unit 2 therefore has a function of binding and immobilizing an antibody to the copolymer via the linker.

For the latter, the unit 2 is more hydrophobic than the unit 1. Thus, the LCST can be adjusted to a lower temperature by increasing the content of the unit 2 in the copolymer.

The copolymer containing 1.0 to 30.0 mol% of the repeating unit represented by formula 2 is likely to have the LCST adjusted to room temperature to 40°C. The binding amount of the antibody is also sufficient for practical use. In the nucleic acid extraction method according to the second embodiment, since the LCST is low, the antibody-copolymer conjugate to which an antigen binds can aggregate more easily (e.g., by simply heating with body temperature) so that the nucleic acid can be extracted.

According to a third embodiment of the nucleic acid extraction method of the present disclosure, in the nucleic acid extraction method according to the first or second embodiment, the repeating unit represented by the above formula 2 is at least one repeating unit selected from the group consisting of a repeating unit represented by formula 4 described later and a repeating unit represented by formula 5 described later.

As described above, the unit 2 has two functions. When the unit 2 is a unit represented by formula 4 and formula 5, its hydrophobicity can be more appropriately adjusted, and as a result, the LCST is likely to be adjusted to a range more easily handled (room temperature to 40°C). The binding to the linker to which an antibody is immobilized becomes easier, and as a result, the convenience and efficiency of the nucleic acid extraction method are further improved.

According to a fourth embodiment of the nucleic acid extraction method of the present disclosure, in the nucleic acid extraction method according to any one of the first to third embodiments, the copolymer further includes a repeating unit represented by formula 6 described later.

The repeating unit represented by formula 6 (unit 6) is more hydrophilic than the unit 2. Thus, the unit 6 has the effect of adjusting the LCST of the copolymer (and the antibody-copolymer conjugate) to a higher temperature. Thus, even when the content of the unit 2 is high, the LCST is likely to be adjusted to a range more easily handled (room temperature to 40°C). Thus, both the binding to an antigen and the ease of handling (convenience) are likely to be achieved at a higher level.

According to a fifth embodiment of the nucleic acid extraction method of the present disclosure, in the nucleic acid extraction method according to any one of the first to fourth embodiments, the content of the repeating unit represented by the above formula 2 is 2.0 to 30.0 mol% when the total of repeating units of the copolymer is defined as 100 mol%.

The copolymer and the antibody-copolymer conjugate achieve both the binding to an antigen and the ease of handling (convenience) at a higher level. As a result, the nucleic acid extraction method according to the fifth embodiment can extract a nucleic acid more reliably and more easily.

According to a sixth embodiment of the nucleic acid extraction method of the present disclosure, in the nucleic acid extraction method according to any one of the first to fifth embodiments, the copolymer has a number average molecular weight of 5000 to 50000.

When the number average molecular weight of the copolymer is 5000 or more, more excellent concentration efficiency is likely to be obtained (in other words, more excellent temperature responsiveness is likely to be obtained). On the other hand, when the number average molecular weight is 50000 or less, a conjugate with an antibody is likely to be formed, or an antigen-antibody reaction is likely to proceed. As a result, the nucleic acid extraction method according to the sixth embodiment using the antibody-copolymer conjugate based on the above copolymer allows an antigen to bind to the antibody-copolymer conjugate more easily and can extract a nucleic acid more efficiently.

According to a seventh embodiment of the nucleic acid extraction method of the present disclosure, in the nucleic acid extraction method according to any one of the first to sixth embodiments, a ratio of the content of the copolymer to the content of the antibody contained in the antibody-linker complex (copolymer/antibody) in the mixture solution is 0.5 to 30.0 on a molar basis.

The copolymer and the antibody-linker complex bind to each other easily and reliably through a click reaction. The copolymer itself has the same LCST as the antibody-copolymer conjugate. Thus, even when the content of the copolymer is lower than that of the antibody-linker complex in the mixture solution, the copolymer that is not involved in binding to the antibody-linker complex can contribute to the aggregation with temperature change (heating) and the extraction of a nucleic acid. In this regard, the lower limit of a numerical range of the copolymer/antibody is preferably 1.0 or more, more preferably 2.0 or more. Suitable forms of the numerical range of the copolymer/antibody will be described later.

According to an eighth embodiment of the nucleic acid extraction method of the present disclosure, in the nucleic acid extraction method according to any one of the first to seventh embodiments, the antibody-copolymer conjugate includes a repeating unit represented by formula 1 described later and a repeating unit represented by formula 7 described later.

The copolymer is typically obtained by binding an antibody-linker complex to a copolymer having a unit 5. A unit 7 based on the unit 5 has a function of adjusting the LCST to a temperature easily handled (room temperature to 40°C). The nucleic acid extraction method according to the eighth embodiment using the antibody-copolymer conjugate therefore allows the antibody-copolymer conjugate to which an antigen binds (or does not bind) and the copolymer to easily aggregate and precipitate and can extract a nucleic acid more easily.

According to a ninth embodiment of the nucleic acid extraction method of the present disclosure, in the nucleic acid extraction method according to any one of the first to eighth embodiments, the heating is heating to increase the temperature of the mixture solution to 20 to 40°C.

The heating of the mixture solution to 20 to 40°C can also be achieved, for example, by warming a container containing the mixture solution with the body temperature of a laboratory technician. The nucleic acid extraction method according to the ninth embodiment can extract a nucleic acid easily without using heating equipment or the like.

According to a tenth embodiment of the nucleic acid extraction method of the present disclosure, in the nucleic acid extraction method according to any one of the first to ninth embodiments, the specimen is a membrane structure having a lipid bilayer.

It has been experimentally confirmed that when the specimen has a lipid bilayer and the nucleic acid contained therein is a target to be extracted, the nucleic acid can be extracted only by, in the absence of a proteolytic enzyme, preparing a mixture solution containing a specimen, a copolymer, and an antibody-linker complex to generate an antibody-copolymer conjugate, and heating the mixture solution to cause aggregation of the antibody-copolymer conjugate (and the copolymer, if included in the mixture solution) (as will be described later). The nucleic acid extraction method according to the tenth embodiment can extract a target nucleic acid more easily, with a very simple procedure, and without using many reagents, compared with conventional methods.

According to an eleventh embodiment of the nucleic acid extraction method of the present disclosure, in the nucleic acid extraction method according to the first to ninth embodiments, the specimen is an enveloped virus.

It has been experimentally confirmed that when the specimen is an enveloped virus, the nucleic acid extraction method of the present disclosure can extract a target nucleic acid more easily, with a very simple procedure, and without using many reagents, compared with conventional methods.

According to a twelfth embodiment of the nucleic acid extraction method of the present disclosure, in the nucleic acid extraction method according to the first to ninth embodiments, the specimen is SARS-CoV-2 virus.

It has been experimentally confirmed that when the specimen is SARS-CoV-2 virus, the nucleic acid extraction method of the present disclosure can extract a target nucleic acid more easily, with a very simple procedure, and without using many reagents, compared with conventional methods.

A nucleic acid amplification method according to a first embodiment of the present disclosure includes: extracting the nucleic acid from the specimen using any one of the first to twelfth embodiments of the nucleic acid extraction method; and amplifying the extracted nucleic acid through a polymerase chain reaction.

The nucleic acid amplification method according to the first embodiment, which includes the step of extracting a nucleic acid by the nucleic acid extraction method described above, achieves labor savings in the entire steps and requires fewer reagents and less equipment, compared with conventional methods.

A nucleic acid extraction kit according to a first embodiment of the present disclosure is a nucleic acid extraction kit to be used for extracting a nucleic acid from at least one specimen selected from the group consisting of cells, extracellular vesicles, and virions, in the absence of a proteolytic enzyme. The nucleic acid extraction kit includes a first agent including a copolymer including a repeating unit represented by formula 1 described later and a repeating unit represented by formula 2 described later, and a second agent including an antibody-linker complex obtained by binding an antibody that binds to the specimen and a linker represented by the above formula 3 via an amide bond.

With the nucleic acid extraction kit according to the first embodiment, which includes the first agent including the copolymer and the second agent including the antibody-linker complex, an antibody-copolymer conjugate can be prepared by mixing the first agent and the second agent when necessary. Since the first agent and the second agent are separated from each other, the preservability is high, and the manipulation is only mixing in use and therefore is easy. The use of the nucleic acid extraction kit according to the first embodiment enables extraction of a nucleic acid in the absence of a protein enzyme and with a simple manipulation.

A polymerase chain reaction (PCR) test kit of the present disclosure is a PCR test kit including the first embodiment of the nucleic acid extraction kit.

With the PCR test kit, a nucleic acid can be easily extracted from a specimen contained in a biological sample or the like and can be amplified, in the absence of a proteolytic enzyme. The use of the present PCR test kit considerably can reduce the time required for a PCR test and the necessary costs.

The nucleic acid extraction method and the like will be described in detail below based on non-limiting embodiments.

In the present specification, a numerical range represented by using "to" means a range including the numerical values described before and after "to" as lower and upper limits.

In the present specification, when there is more than one substituent or linking group or the like (hereinafter referred to as "substituent or the like") denoted by a specific sign, or when multiple substituents or the like are specified simultaneously, this means that the substituents or the like may be the same or different from each other. This is also applicable to the specification of the number of substituents or the like.

When multiple substituents or the like are in close proximity (in particular adjacent), they may be linked or fused together to form a ring, unless otherwise specified.

In the present specification, the substituents or the like not specified as substituted or unsubstituted may have additional substituents to the extent that the desired effect is not impaired. This is also applicable to compounds that are not specified as substituted or unsubstituted.

In the present specification, "virion" means a virus particle of an infectious virus. An infectious virus can replicate in cells of bacteria, plants, and animals including humans (collectively also referred to as "hosts"). The virion in one form is a metabolically inert infectious pathogen with a diameter of 20 to 300 nm and includes a nucleic acid (RNA or DNA) core and a protein outer shell. Furthermore, an enveloped virus with an envelope including a lipid bilayer is also included in the above.

Examples of the infectious virus include influenza viruses (avian influenza virus, equine influenza virus, swine influenza virus, canine influenza virus, feline influenza virus, and human influenza virus), human immunodeficiency virus (HIV), flaviviruses (e.g., hepatitis virus, dengue virus, Zika virus, etc.), human papillomavirus (HPV), bovine papillomavirus, herpesvirus (e.g., HSV-I, HSV-II, CMV, and VZV), rhinoviruse, coronaviruses (e.g., SARS-CoV-2, SARS coronavirus, MERS coronavirus, etc.), enterovirus, polyomavirus, respiratory syncytial virus (RSV), hepatitis B virus, hepatitis C virus, rotavirus, measles virus, mumps virus, rubella virus, varicella virus, human metapneumovirus, Ebola virus, Marburg virus, Alphavirus (e.g., Chikungunya virus, Ross River virus, Sindbis virus, Mayaro virus, etc.), porcine epidemic diarrhea, porcine reproductive and respiratory syndrome virus, and foot-and-mouth disease virus.

In the present specification, "extracellular vesicle" means a particle-like structure that is released from a cell to the extracellular environment by any mechanism. "Extracellular vesicle" includes exosome, microvesicle, and apoptotic body. Extracellular vesicles may contain proteins, nucleic acids, lipids, and other molecules derived from their host cells, and extracellular vesicles containing nucleic acids are preferred as the specimen in the present nucleic acid detection method. The extracellular vesicles may be derived from various cells, such as red blood cells, white blood cells, cancer cells, stem cells, dendritic cells, and macrophages.

In the present specification, "cell" can be any of prokaryotic cell or eukaryotic cell and is not particularly limited. Examples of the cell include bacteria, archaea bacteria, yeast, plant cells, insect cells, and animal cells (e.g., human cells, non-human cells, non-mammalian vertebrate cells, invertebrate cells, etc.).

In the present specification, "specimen" is at least one selected from the group consisting of cells, extracellular vesicles, and virions, and virions are preferred. The specimen is preferably a membrane structure having a lipid bilayer (cell, vesicle, liposome, enveloped virus, etc.), more preferably an enveloped virus, and further preferably SARS-CoV-2 virus.

The specimen may be included in a sample obtained by culture or the like or may be included in a biological sample collected from animals including humans, plants, and the like.

Examples of the sample obtained from humans may include lower respiratory tract-derived samples such as sputum, tracheal aspirate, and bronchoalveolar lavage fluid; rhinopharyngeal swab such as nasopharyngeal swab and pharyngeal swab; saliva; serum; whole blood; urine; stool; and autopsy tissue.

In the present specification, "antibody" and an antibody residue represented by "Ab" mean an immunoglobulin molecule, or a portion (fragment) thereof, that has the ability to bind to an epitope of an antigen molecule. The "antibody" includes a monoclonal antibody, a polyclonal antibody, a multispecific antibody (e.g., bispecific antibody), and an antibody fragment.

In the present specification, "nucleic acid" means a deoxyribonucleic acid (DNA) molecule and an RNA molecule and may be single-stranded or double-stranded.

In the present specification, "proteolytic enzyme" means an enzyme that has the function of recognizing a specific site of an amino acid sequence constituting a protein, cleaving the bond, and breaking down into amino acids or peptides. Proteolytic enzymes are also called "proteases" and "proteinases".

Proteolytic enzymes include, but not limited to, proteolytic enzymes used for extraction and/or purification of nucleic acids. Conventionally, nonspecific proteolytic enzymes such as proteinase K (EC3.4.21.64, etc.), protease (pronase), trypsin, and subtilisin are often used in extraction of nucleic acids.

Nucleic acid extraction methods performed "in the presence" of proteolytic enzymes (in other words, nucleic acid extraction methods using proteolytic enzymes) are well known to those skilled in the art, for example, in Japanese Unexamined Patent Application Publication Nos. 2004-215676, 2006-087394, and 2006-061041, Japanese Unexamined Patent Application Publication (Translation of PCT Application) No. 2011-525806, and International Publication WO2017/200249 and WO2017/200249.

As used herein "in the absence of a proteolytic enzyme" means that extraction is performed in an environment (extraction reaction system) where no proteolytic enzyme is used. Typically, it means that nucleic acid extraction is performed without the intentional use of a proteolytic enzyme. This does not preclude unintentional inclusion of a proteolytic enzyme derived from, for example, a specimen in the extraction reaction system, but it is preferable that the extraction reaction system does not include a proteolytic enzyme.

In this specification, "carrier nucleic acid" means a compound that has a function of improving the efficiency of nucleic acid extraction from a specimen when added to a sample. Examples of the carrier nucleic acid include polyadenylic acid (homopolymer). In general, carrier nucleic acids are often used for nucleic acid extraction from samples with a low concentration of nucleic acids included in pathogens and/or samples.

Commercially available nucleic acid extraction kits, such as "QIAamp Viral RNA Mini Kit", already contain carrier RNA.

### [Nucleic acid extraction method]

An embodiment of the nucleic acid extraction method (hereinafter also referred to as "the present nucleic acid extraction method") includes, in the absence of a proteolytic enzyme (and without using a carrier nucleic acid): preparing a mixture solution containing at least one specimen selected from the group consisting of cells, extracellular vesicles, and virions (hereinafter also simply referred to as "specimen"), a copolymer (hereinafter also referred to as "specific copolymer") including a repeating unit represented by formula 1 described later and a repeating unit represented by formula 2 described later, and an antibody-linker complex obtained by binding an antibody that binds to the specimen and a linker represented by formula 3 described later via an amide bond, and generating an antibody-copolymer conjugate; and heating the above mixture solution to allow the antibody-copolymer conjugate to aggregate, and extracting a nucleic acid encapsulated in the specimen.

### <Step S1>

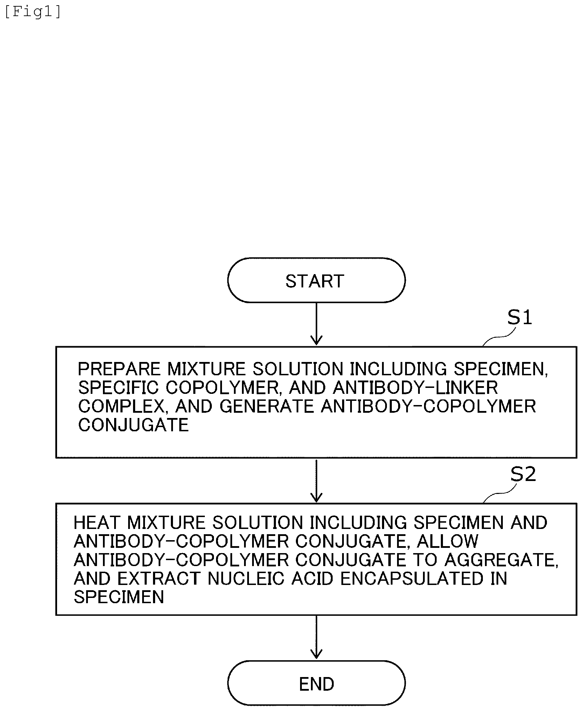

Fig. 1 is a flowchart illustrating a procedure of the present nucleic acid extraction method. First, as step S1, a mixture solution containing the specimen, the specific copolymer, and the antibody-linker complex is prepared to generate the antibody-copolymer conjugate. In this case, it is not necessary to perform treatment of impurities with a proteolytic enzyme, but of course, it may be done.

As will be detailed later, the specific copolymer has a repeating unit represented by formula 1 described later (hereinafter also referred to as "unit 1") and a repeating unit represented by formula 2 described later (hereinafter also referred to as "unit 2"). A polymer constituted of the unit 1 alone shows temperature responsiveness of a lower critical solution temperature (LCST) of 32°C in water. The specific copolymer including the unit 1 has temperature responsiveness.

The unit 2 has one site (click reaction site) including cyclic alkyne (alkynylene group) per repeating unit and can easily form a complex by binding to an azido group of a linker described later through a click reaction.

In other words, the specific copolymer having both the unit 1 and the unit 2 has both the function of bonding to a linker through a click reaction and the function of changing solubility in water upon heating (specifically, the solubility decreases to cause aggregation).

On the other hand, the linker represented by formula 3 described later has an azido group in the molecule and an active ester group. The active ester means a carboxylic acid derivative that can react with an amino group. The linker has an N-hydroxysuccinimide group as the active ester group.

Since the linker has an N-hydroxysuccinimide group, it can bind to an amino group of the antibody to form a complex (antibody-linker complex).

In this step, in the mixture solution containing the specific copolymer having the above characteristics and the antibody-linker complex, the click reaction site of the specific copolymer reacts with the azido group of the antibody-linker complex to generate the antibody-copolymer conjugate.

### (Specific copolymer)

The specific copolymer is a copolymer including a repeating unit represented by the following formula 1 (unit 1) and a repeating unit represented by the following formula 2 (unit 2).

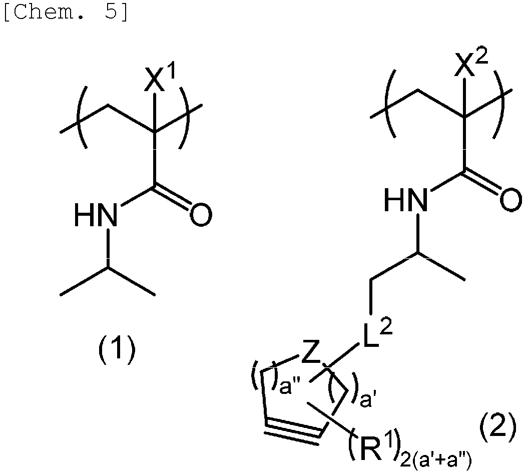

### - Unit 1

A polymer constituted of the unit 1 alone shows temperature responsiveness of a lower critical solution temperature (LCST) of 32°C in water. The specific copolymer has temperature responsiveness by including the unit 1. Specifically, the solubility in water changes with temperature change.

The content of the unit 1 included in the specific copolymer is not particularly limited and is typically preferably 1 to 99 mol% when the total of repeating units is defined as 100 mol%. In particular, from the viewpoint of more sensitive temperature responsiveness of the specific copolymer and/or from the viewpoint of easily controlling the LCST within a temperature range easily handled in the manipulation of nucleic acid extraction (specifically, easily controlling the LCST to room temperature to about 40°C), the content of the unit 1 in the specific copolymer is preferably more than 50 mol%, more preferably 60 mol% or more, and preferably 97 mol% or less.

The content of the unit 1 in the specific copolymer is preferably 1 to 99 mol%; more than 50 mol% and 99 mol% or less; 60 to 99 mol%; 1 to 97 mol%; more than 50 mol% and 97 mol% or less; or 60 to 97 mol%.

In formula 1, X¹ represents a hydrogen atom or a linear or branched alkyl group having 1 to 6 carbon atoms and, from the viewpoint of more sensitive temperature responsiveness of the specific copolymer, is preferably a hydrogen atom or a linear alkyl group having 1 to 4 carbon atoms, more preferably a hydrogen atom or a methyl group.

The unit 1 is preferably, but not limited to, a unit based on a monomer represented by the following formula 1'.

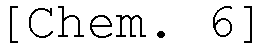

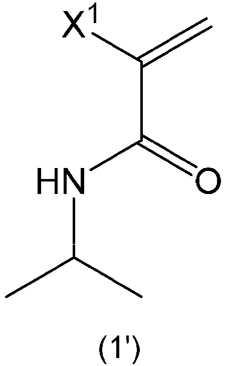

In formula 1', X¹ is synonymous with X¹ in formula 1, and the suitable forms thereof are also the same. The monomer represented by formula 1' may be synthesized by, for example, a known method such as the method described in Synthesis Examples described later or may be a commercially available product.

### - Unit 2

The specific copolymer includes the unit 2 represented by formula 2. The unit 2 has one site (click reaction site) including cyclic alkyne (alkynylene group) per repeating unit and can easily form a complex by binding to an azido group of a linker described later through a click reaction. A protein can be immobilized to the specific copolymer via a linker by binding the linker to the antibody in advance. In other words, an antibody-copolymer conjugate can be produced.

The produced conjugate aggregates and precipitates at a temperature of the LCST or higher due to the temperature responsiveness derived from the specific copolymer. By using this, a corresponding antigen can be concentrated, and a nucleic acid thus released can be extracted and concentrated.

The content of the unit 2 in the specific copolymer is not particularly limited and is typically preferably 1 to 99 mol% when the total of repeating units of the specific copolymer is defined as 100 mol%.

In particular, in terms of achieving more excellent effects of the present invention, the content is preferably 1.0 mol% or more, more preferably 2.0 mol% or more, and preferably 30 mol% or less, more preferably 20 mol% or less.

The content of the unit 2 in the specific copolymer is preferably 1.0 to 99.0 mol%, 2.0 to 99.0 mol%, 1.0 to 30.0 mol%, 2.0 to 30.0 mol%, 1.0 to 20.0 mol%, or 2.0 to 20.0 mol%.

The unit 2 is typically more hydrophobic than the unit 1 or other repeating units described later. The LCST therefore can be reduced by increasing the content of the unit 2 in the specific polymer.

The higher the content of the unit 2, the easier the introduction of an antibody. On the other hand, from the viewpoint of adjusting the LCST to 20 to 40°C, the content of the unit 2 is preferably 1.0 mol% or more, more preferably 2.0 mol% or more, and preferably 30 mol% or less, more preferably 20 mol% or less.

From the viewpoint of adjusting the LCST to 20 to 40°C, the content is preferably 1.0 to 30.0 mol%, 1.0 to 20.0 mol%, 2.0 to 30.0 mol%, or 2.0 to 20.0 mol%.

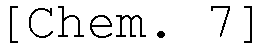

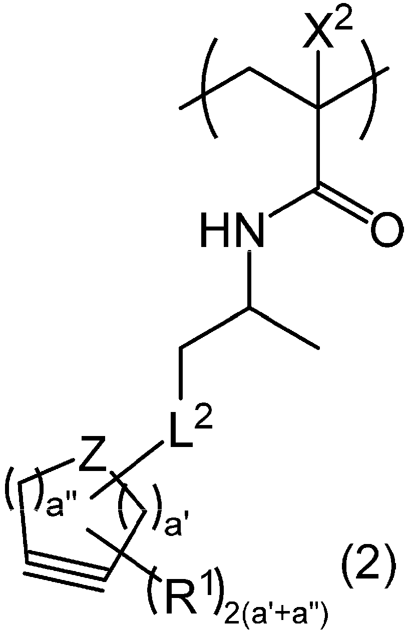

In formula 2, X² represents a hydrogen atom or a linear or branched alkyl group having 1 to 6 carbon atoms and, from the viewpoint of more sensitive temperature responsiveness of the specific copolymer, is preferably a hydrogen atom or a linear alkyl group having 1 to 4 carbon atoms, more preferably a hydrogen atom or a methyl group.

In formula 2, L² represents a divalent group. The divalent group is not particularly limited and is preferably at least one selected from the group consisting of -O-, -S-, -C(O)-, -C(O) O-, -OC(O)O-, -NR^{A}- (R^{A} is a hydrogen atom or a monovalent substituent), a linear, branched, or cyclic aliphatic hydrocarbon group having 1 to 20 carbon atoms, a monocyclic or condensed aromatic hydrocarbon group having 6 to 20 carbon atoms, and a combination thereof.

In particular, from the viewpoint of obtaining a copolymer having more excellent effects of the present invention, one group selected from the group consisting of -O-, -C(O)-, -NR^{A}-, a linear alkylene group having 1 to 10 carbon atoms, and a combination thereof is more preferred, at least one group selected from the group consisting of - O-, -C(O)-, -NR^{A}-, and a linear alkylene group having 1 to 10 carbon atoms is further preferred, and -O-, -C(O)-, or - NH- is particularly preferred.

In formula 2, R¹ is selected from the group consisting of a hydrogen atom, a halogen atom, -OR⁵, -NO₂, -CN, -S(O)₂R⁵, an alkyl group having 1 to 24 carbon atoms, an alkenyl group having 2 to 24 carbon atoms, and a (hetero)aryl group having 6 to 24 carbons atoms, where two or more R¹s are optionally bonded to each other to form a ring, and R⁵ represents a group selected from the group consisting of hydrogen, a halogen atom, an alkyl group having 1 to 24 carbon atoms, and a (hetero)aryl group having 6 to 24 carbon atoms. A plurality of R¹s may be the same or different from each other.

In formula 2, Z represents a group selected from the group consisting of C(R¹)₂, O, S, and NR¹. When a divalent group represented by L² is bonded to Z, Z is a group from which one of the hydrogen atom, halogen atom, or carbon atom of C(R¹)₂ or NR¹ is removed. More specifically, Z is a structure represented by =C(R¹)R^{X}-L²- or a structure represented by =N-R^{x}-L²-. Here, R^{X} represents a single bond, or an alkylene group (having 1 to 24 carbon atoms), an alkenylene group (having 2 to 24 carbon atoms), an alkynylene group (having 2 to 24 carbon atoms), or an arylene group (having 6 to 24 carbon atoms) optionally having a heteroatom.

In formula 2, a' is an integer of 0 to 8, a" is an integer of 0 to 8, and the sum of a' and a" is less than 10.

In terms of obtaining more excellent effects of the present invention, the unit 2 is preferably a unit represented by the following formula 2^{a}.

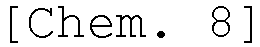

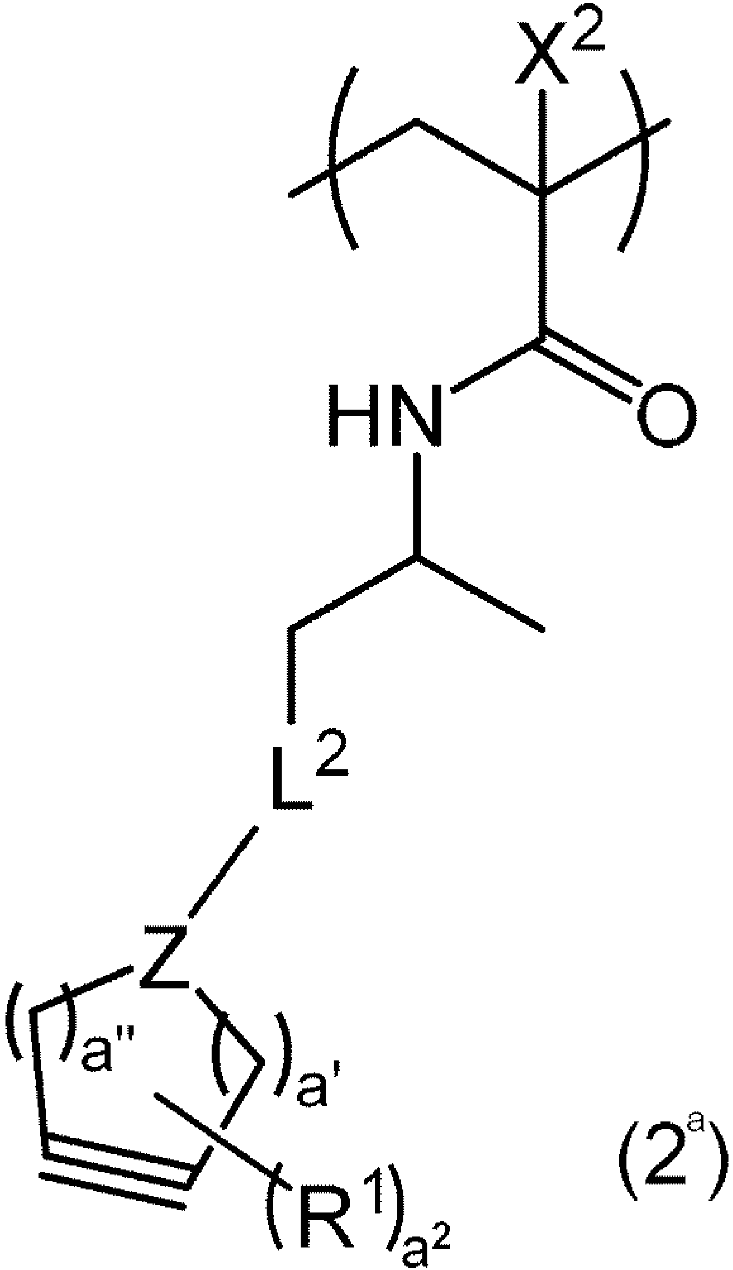

In formula 2^{a}, X², L², R¹, a', and a" are synonymous with the respective symbols in formula 2, and the suitable forms thereof are also the same. In formula 2^{a}, Z represents CR¹ or a nitrogen atom, and a² is a number equal to or less than a' + a". R¹ is synonymous with R¹ in formula 2, and the suitable forms thereof are also the same.

The unit 2 preferably has a structure (click reaction site) represented by, for example, the following formulae C in which "*" represents the binding site with L².

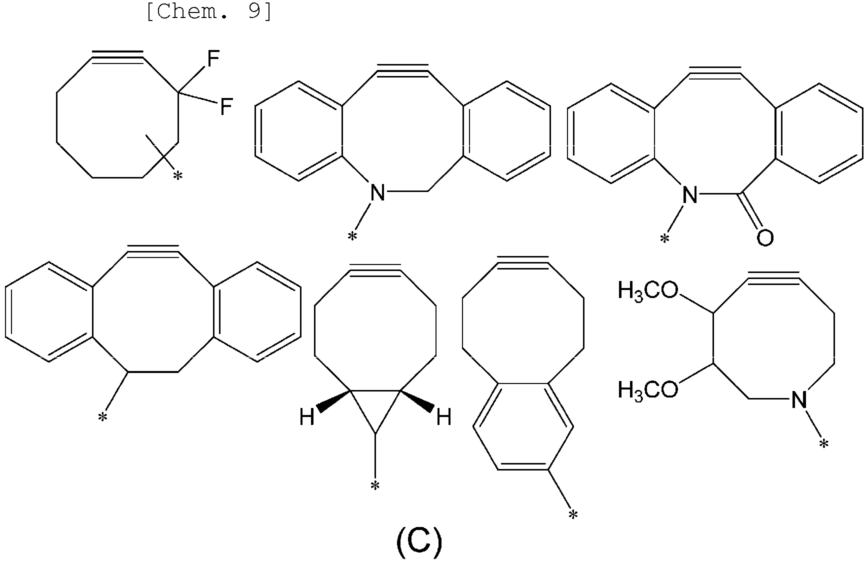

The unit 2 preferably has a structure represented by any of the above formulae C as a site (monovalent group) that is bonded at the position of "*". The * in the monovalent group represented by formulae C is bonded to the wave line in the following formula.

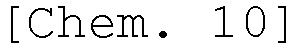

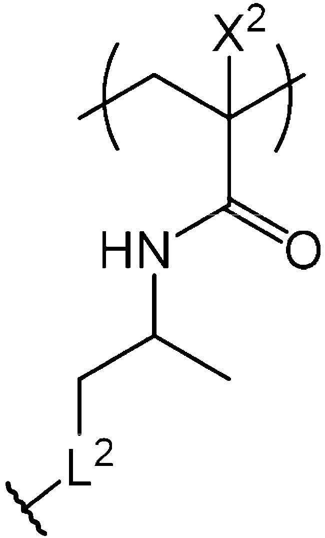

In the above formula, X² and L² are synonymous with the groups represented by the same symbols in formula (2), and the suitable forms are also the same.

From the viewpoint of obtaining a copolymer having more excellent effects of the present invention, the unit 2 is preferably at least one selected from the group consisting of units represented by the following formulae.

In the following formulae, L²¹ represents a divalent group and is synonymous with L² in formula 2, and the suitable forms thereof are also the same. X² is synonymous with X² in formula 2, and the suitable forms thereof are also the same.

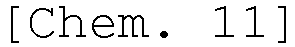

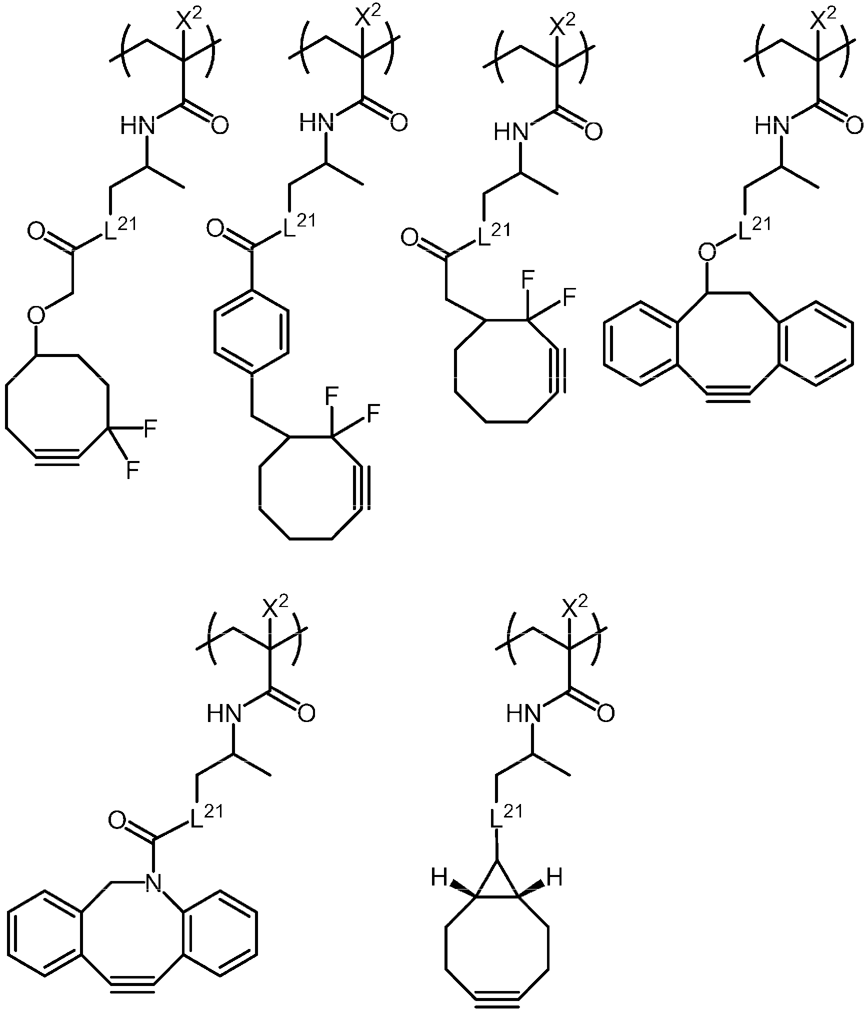

In particular, from the viewpoint of obtaining a copolymer having more excellent effects of the present invention, the unit 2 is preferably a unit 4 or a unit 5 represented by the following formula 4 or 5.

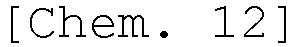

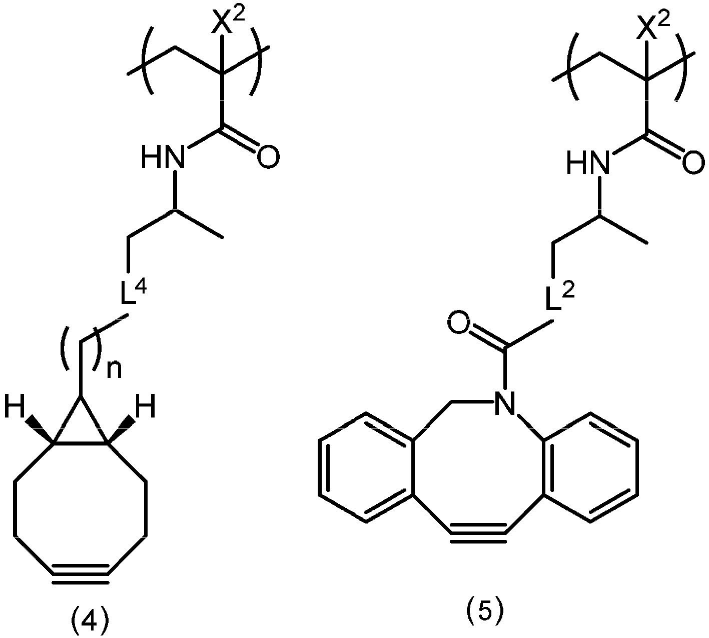

In formula 4 and formula 5, X² represents a hydrogen atom or a linear or branched alkyl group having 1 to 6 carbon atoms; in formula 4, L⁴ is one group selected from the group consisting of -O-, -S-, and -NR^{B}-, where R^{B} represents a hydrogen atom or an alkyl group having 1 to 6 carbon atoms, n represents an integer of 1 to 10; and in formula 5, L² represents a divalent group, and the suitable forms thereof are the same as those of the divalent group of L² in formula 2.

The method for synthesizing the unit 2 is not particularly limited, and although a known synthesis method can be used, a method for obtaining the unit 2 by binding a precursor compound to a unit 2' represented by the following formula 2' is preferable in that the unit 2 can be obtained more easily.

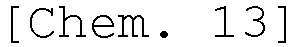

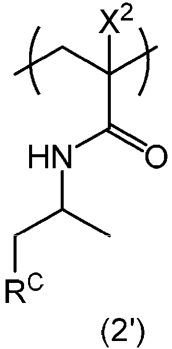

In formula 2', R^{C} is a reactive substituent. Specifically examples include a hydroxy group, an amino group, a carboxy group, a glycidyl group, an epoxy group, a glycidyl ether group, a mercapto group, a hydroxysuccinimide ester, and maleimide, and a hydroxy group is preferable. X² is synonymous with X² in formulae 4 and 5.

The precursor compound may be, for example, a compound including a click reaction site and a group that can react with the R^{C}, and a commercially available product may be used, or a product synthesized by a known method may be used. In particular, when a precursor compound including a structure (click reaction site) represented by any of the formulae C above and a carboxy group is used, the specific copolymer can be synthesized more easily.

As the method for synthesizing a precursor compound, for example, when the click reaction site is difluorinated cyclooctyne, the method described in the schemes 1 and 2 of J. Am. Chem. Soc. 2008, 130, 34, 11486-11493 can be used. When the click reaction site is dibenzoazacyclooctyne, the method described in the scheme 1 of Chemical Communications (2010), 46(1), 97-99 can be used.

As the precursor compound, a compound obtained by attaching a substituent such as an amino group, a hydroxy group, or a carboxy group to a click reaction site such as bicyclo[6.1.0]nonyne (BCN) or dibenzocyclooctyne (DBCO), and a compound esterified with maleimide or hydroxysuccinimide, which are commercially available, can also be used.

In particular, the unit 2' is preferably a unit 6 represented by the following formula 6 in that the unit 2 can be obtained more easily. In this case, a precursor compound including a carboxy group and a structure represented by any of the formulae C may be typically used.

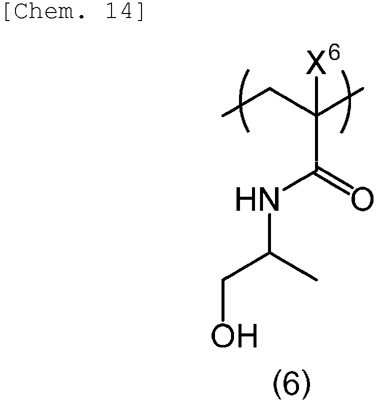

In synthesis of the specific copolymer, if unreacted unit 6 is present, in other words, if the specific copolymer includes the unit 1, the unit 2, and the unit 6, the LCST can be adjusted to a higher temperature. Since the unit 6 is more hydrophilic than other repeating units, the LCST can be adjusted to a higher temperature by increasing the content of the unit 6.

In the above formula 6, X⁶ represents a hydrogen atom or a linear or branched alkyl group having 1 to 6 carbon atoms and, from the viewpoint of more sensitive temperature responsiveness of the copolymer, is preferably a hydrogen atom or a linear alkyl group having 1 to 4 carbon atoms, and more preferably a hydrogen atom or a methyl group.

The content of the unit 6 in the specific copolymer is not particularly limited and is preferably 0 to 15 mol% when the total of repeating units of the specific copolymer is defined as 100 mol%.

The molecular weight of the specific copolymer is not particularly limited and is typically, as a number average molecular weight, preferably 2000 to 100000, more preferably 5000 to 50000, and further preferably 10000 to 30000.

When the number average molecular weight is 5000 or more, more excellent concentration efficiency is likely to be obtained (in other words, more excellent temperature responsiveness is likely to be obtained). On the other hand, when the number average molecular weight is 50000 or less, a conjugate with an antibody is likely to be formed, or an antigen-antibody reaction is likely to proceed.

In particular, when an antibody-copolymer conjugate produced by the obtained specific copolymer is used, the number average molecular weight is preferably 15000 or more, more preferably 20000 or more, in that more excellent concentration efficiency can be obtained. In general, many antibodies are highly hydrophilic, and in such a case, a copolymer having more excellent concentration efficiency is necessary. When the number average molecular weight of the copolymer is within the above numerical range, excellent concentration efficiency is likely to be obtained.

Based on the above, the molecular weight of the specific polymer is preferably 2000 to 100000, 5000 to 50000, 10000 to 30000, 15000 to 100000, 15000 to 50000, 15000 to 30000, 20000 to 100000, 20000 to 50000, or 20000 to 30000.

### (Other units)

The specific copolymer may include a repeating unit other than the above. Examples of the repeating unit other than the above include repeating units based on, for example, N-cyclopropylacrylamide (LCST = 46°C), N-n-propylacrylamide (LCST = 22°C), N-tetrahydrofurfurylacrylamide (LCST = 28°C), N-ethoxyethylacrylamide (LCST = 35°C), N-methyl-N-ethylacrylamide (LCST = 56°C), N-methyl-N-isopropylacrylamide (LCST = 23°C), N-methyl-N-isopropylacrylamide (LCST = 23°C), N-methyl-N-n-propylacrylamide (LCST = 20°C), N,N-diethylacrylamide (LCST = 32°C), N-cyclopropylmethacrylamide (LCST = 59°C), N-isopropylmethacrylamide (LCST = 44°C), N-n-propylmethacrylamide (LCST = 28°C), and N-tetrahydrofurfurylmethacrylamide (LCST = 35°C).

### (Method for manufacturing specific copolymer)

The method for manufacturing the specific copolymer is not particularly limited and, from the viewpoint of being able to manufacture the specific copolymer more easily, the method preferably includes the following step (1) and step (2) in this order.

Step (1): a step of copolymerizing monomers represented by the following formula 1' and formula 3' to obtain a copolymer (precursor of the specific copolymer).

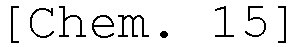

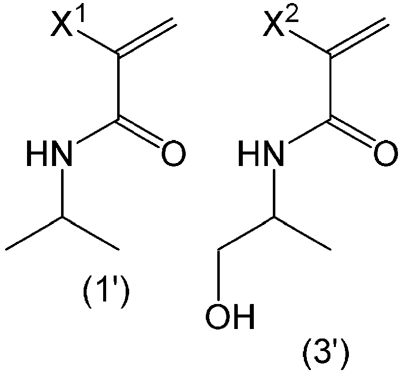

In the above formulae 1' and 3', X¹ and X² are synonymous with X¹ in formula 1 and X² in formula 2', respectively, and the suitable forms thereof are also the same.

The method for copolymerizing the monomers is not particularly limited, and it is preferable to use living polymerization such as living radical polymerization, living anionic polymerization, and living cationic polymerization. In particular, from the viewpoint of obtaining a copolymer (or a precursor thereof) more easily, living radical polymerization is preferable.

The living radical polymerization is based on that equilibrium between a small amount of propagating radical (free radical) species and a large amount of dormant species is rapidly established in a growth reaction by application of heat, light, a metal catalyst, or the like. Various types of living radical polymerization have been proposed.

Examples include an atom transfer radical polymerization (ATRP) method using halogenated alkyl as a dormant, a reversible addition fragmentation chain transfer (RAFT) method using thioesters as a dormant, and a nitroxide mediated polymerization (NMP) method using alkoxyamine as a dormant.

The RAFT method is a method for polymerizing a vinyl-based monomer by adding a chain transfer agent having a high chain-transfer constant called a RAFT agent to a usual radical polymerization system. Thioesters can be used as the RAFT agent.

The amount of the RAFT agent can be selected as appropriate depending on the molecular weight of the target copolymer. In other words, since the RAFT agent binds to the terminal of each copolymer, for example, when the target is a 100-mer copolymer, the RAFT agent may be used in an amount of 0.1 to 3 mol% with respect to 100 mol% of the monomer.

The radical polymerization initiator to be used for the RAFT polymerization is not particularly limited and may be appropriately selected from known initiators such as an azo compound, a peroxide, and a redox-type initiator.

Examples of the azo compound include 2,2'-azobisisobutyronitrile (AIBN), 2,2'-azobis-2,4-dimethylvaleronitrile, 2,2'-azobis(2-methylpropionamidine) dihydrochloride, and 4,4'-azobis(4-cyanovaleric acid).

The amount of the polymerization initiator is generally preferably 0.1 to 50 mol% with respect to 1 mol of the RAFT agent. The reaction temperature of the RAFT method is determined by the radical polymerization initiator to be used, and is generally 40°C to 150°C in many cases. Polymerization is performed under atmospheric pressure in many cases, but can also be performed under pressure.

The RAFT method can be performed in the absence of a solvent, but can also be performed in the presence of a solvent. The solvent that is used as necessary is not particularly limited and any known solvent can be used. The reaction can also be performed in water, and the reaction also proceeds in emulsion polymerization. The emulsifier used in this case can be a nonionic emulsifier, a cationic emulsifier, and an anionic emulsifier that can be used in general emulsion polymerization.

Step (2): a step of obtaining a specific copolymer by reacting the obtained copolymer (or a precursor thereof) and a precursor compound represented by formula 10.

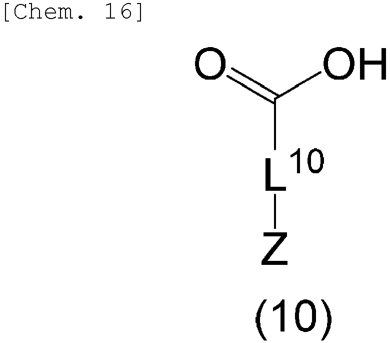

In formula 10, Z is a group including a click reaction site (e.g., cyclic alkyne) and is preferably a group selected from the groups represented by formulae C already explained. In formulae C, * is the binding position with L¹⁰.

L¹⁰ represents a divalent group and is synonymous with L² in formula 2, and the suitable forms thereof are also the same.

A desired specific copolymer is synthesized by forming an ester bond (condensation) between a hydroxy group of the copolymer (precursor) and a carboxy group of the precursor compound represented by formula 10.

The method for forming an ester bond is not particularly limited, and examples thereof include a method by a condensation reaction of a copolymer (precursor) and a precursor compound in the presence of a condensing agent, a catalyst, and a solvent at 0 to 150°C (preferably 0 to 100°C) for 30 minutes to 24 hours.

As the condensing agent, for example, triphenyl phosphite, N,N'-dicyclohexylcarbodiimide, 1-ethyl-3-(3-dimethylaminopropyl)carbodiimide hydrochloride, N,N'-carbonyldiimidazole, dimethoxy-1,3,5-triazinylmethyl morpholinium, O-(benzotriazol-1-yl)-N,N,N',N'-tetramethyluronium tetrafluoroborate, O-(benzotriazol-1-yl)-N,N,N',N'-tetramethyluronium hexafluorophosphate, and diphenyl(2,3-dihydro-2-thioxo-3-benzoxazolyl)phosphonate can be used. In particular, N,N'-dicyclohexylcarbodiimide is preferable. In this case, N,N-dimethyl-4-aminopyridine or the like can be used as a catalyst, and dichloromethane or the like can be used as a solvent.

### (Antibody-linker complex)

The antibody-linker complex is a compound obtained by binding an antibody that binds to the specimen and a linker represented by the following formula 3 via an amide bond.

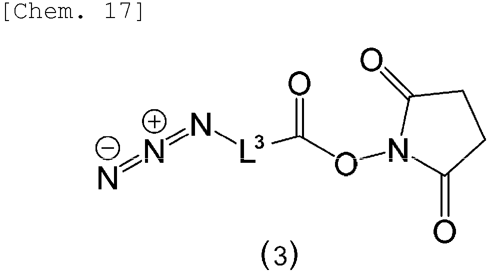

In formula 3, L³ represents a divalent hydrocarbon group optionally having a heteroatom.

The divalent hydrocarbon group is not particularly limited and is preferably a (poly)oxyalkylene group (the number of carbon atoms of the alkylene group is preferably 1 to 6) and a linear, branched, or cyclic hydrocarbon group having 1 to 20 carbon atoms, or the like, and more preferably a polyoxyalkylene group. The repeating number of the polyoxyalkylene group is preferably 2 to 10 and more preferably 2 to 8.

The linker represented by formula 3 includes an N-hydroxysuccinimidyl ester (NHS ester) and can form an amide bond with a primary amine (e.g., lysine residue) of a protein. In other words, the linker binds to -NH₂ of an antibody to form a complex. On the other hand, the linker has an azido group and binds to an alkynylene group (click reaction site) of the specific copolymer to form a triazole ring.

The method for producing the antibody-linker complex is not particularly limited. The linker represented by formula 3 may be dissolved in an organic solvent or the like (e.g., a nonprotic polar solvent such as dimethyl sulfoxide), and the resulting solution may be added to a solution in which an antibody is dispersed in a buffer solution (e.g., carbonate buffer solution) containing a buffering agent if necessary. The reaction temperature is not particularly limited and is preferably 1 to 20°C and more preferably 1 to 10°C. The reaction time is not particularly limited and is preferably 1 to 24 hours.

The addition ratio between the antibody and the linker during manufacturing of the antibody-linker complex is not particularly limited, and the ratio (molar ratio, active ester group/amino group) of the active ester group of the linker to the content of the amino group of the antibody is preferably 0.1 to 500 on an amount of substance basis.

The amount of the linker added per mole of the antibody is not particularly limited and is preferably 0.01 to 5000 moles, more preferably 1 to 2000 moles, further preferably 50 to 200 moles.

The antibody to be used is not particularly limited as long as it binds to a specimen (a protein thereof) and has a primary amino group. For example, when the specimen is SARS-CoV-2 virus, the antibody may be against S (spike) protein or N (nucleocapsid) protein. The antibody may be any of a monoclonal antibody, a polyclonal antibody, a multispecific antibody, and an antibody fragment.

### (Antibody-copolymer conjugate)

In the antibody-copolymer conjugate, the click reaction site of the specific copolymer and the azido group of the antibody-linker complex are bonded to form a triazole ring structure.

Examples of the antibody-copolymer conjugate include a compound having a repeating unit represented by the following formula 1 and formula 7.

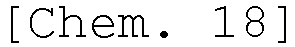

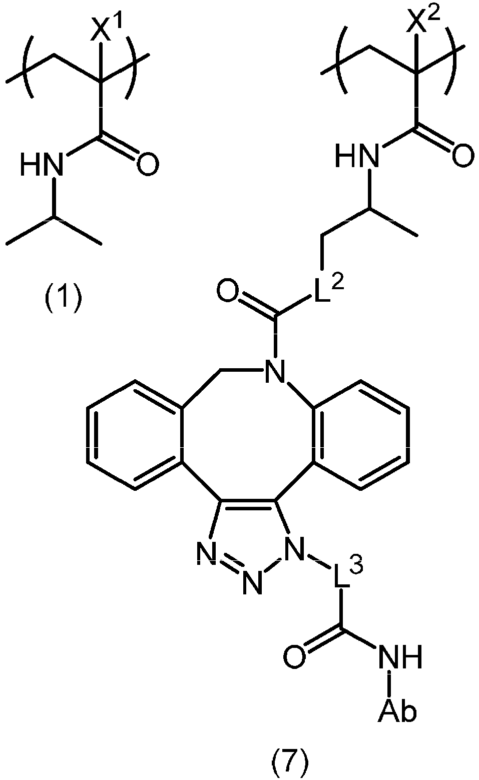

In formula 1, X¹ is synonymous with X¹ of the specific copolymer, and the suitable forms thereof are also the same. In formula 7, X² represents a hydrogen atom or a linear or branched alkyl group having 1 to 6 carbon atoms and is synonymous with X² in formula 2, and the suitable forms thereof are also the same.

L² represents a divalent group and is synonymous with L² in formula 2, and the suitable forms thereof are also the same. L³ represents a divalent hydrocarbon group optionally having a heteroatom and is synonymous with L³ in formula 3, and the suitable forms thereof are also the same.

Ab represents an antibody residue. In other words, it represents a state in which an amide bond is formed by the primary amine of the antibody and the NHS ester of the linker to immobilize the antibody to the specific copolymer via the linker.

In the state above, the antibody and the unit 7 bind to each other at a 1:1 ratio. However, the antibody-copolymer conjugate is not limited to the above state, and a state in which a plurality of primary amines of the antibody bind to alkynylene groups of the specific copolymer, respectively, via a linker, that is, a structure like a crosslinked structure centered on the antibody molecule, may be formed.

The antibody-copolymer conjugate may include, in addition to the repeating units above, the unit 2 represented by formula 2. In other words, the conjugate may include an unreacted unit 2. As one embodiment, since the molecule of the specific copolymer is smaller than the molecule of the antibody, it may be difficult for the antibody to bind to each of binding sites of the specific copolymer even when the specific copolymer has a plurality of binding sites. In this case, an unreacted unit 2 may remain in the antibody-copolymer conjugate.

The ratio between the antibody and the specific copolymer is not particularly limited and, as one embodiment, the amount of the specific copolymer is preferably 0.1 to 50 moles with respect to 1 mole of the antibody.

The antibody-copolymer conjugate may include another unit (unit 5) or the like that may be included in the specific copolymer, and the preferred range of the content of each unit is the same as the preferred range of the unit in the specific copolymer.

The antibody is not particularly limited, and a known antibody can be used. In particular, an anti-SARS-CoV-2 antibody is preferable, and, for example, an anti-SARS-CoV-2 (COVID19) nucleocapsid (protein) antibody and an anti-SARS-CoV-2 spike (protein) antibody are more preferable.

The content ratio of the content of the specific copolymer to the content of the antibody in the antibody-copolymer conjugate on a molar basis is not particularly limited and is preferably 0.1 to 100.0, 0.5 to 70.0, 0.5 to 50.0, 0.5 to 30.0, 1.0 to 50.0, 1.0 to 40.0, 2.0 to 100.0, 2.0 to 50.0, 5.0 to 100.0, 5.0 to 50.0, 10.0 to 100.0, 10.0 to 50.0, or 10.0 to 30.0.

The method for producing the antibody-copolymer conjugate is not particularly limited. The specific copolymer and the antibody-linker complex may be mixed and stirred in water containing a buffering agent if necessary. The reaction temperature is not particularly limited and is preferably 1 to 20°C and more preferably 4 to 10°C. The reaction time is not particularly limited and is preferably 1 to 24 hours.

Returning to the flow in Fig. 1, the method for preparing the mixture solution in step S1 is not particularly limited and includes mixing the biological sample, the antibody-linker complex, and the specific copolymer. When the biological sample is liquid, the other components may be added to the biological sample. The order in which the antibody-linker complex and the specific copolymer are added is not particularly limited. The specific copolymer may be added after the antibody-linker complex is added, the antibody-linker complex may be added after the specific copolymer is added, or the antibody-linker complex and the specific copolymer may be added at the same time.

The amount of the antibody-specific copolymer conjugate added to the biological sample is not particularly limited. For example, the amount of the antibody-specific copolymer conjugate to be added is preferably 0.1 mg or more, more preferably 0.5 mg or more, further preferably 1.0 mg or more, per 1 mg of the specimen (antigen protein). The upper limit is not particularly limited and is generally 5.0 mg or less.

More specifically, the amount of the antibody-specific copolymer conjugate to be added is preferably 0.1 to 10.0 mg per 1 mL of a liquid medium containing a specimen (e.g., including saliva, etc.).

As described above, the amount of the antibody-specific copolymer conjugate to be added to the biological sample is preferably 0.1 to 5.0 mg, 0.5 to 5.0 mg, or 1.0 to 5.0 mg per 1 mg of a specimen (antigen protein).

In this case, the mixture solution may further contain the specific copolymer in a free state (not forming a conjugate with an antibody). The addition of the specific copolymer makes the response to heat more sensitive and enables more efficient concentration of the specimen. The amount of the specific copolymer to be added is not particularly limited and is preferably 0.1 to 20 times the content of the specific copolymer contained in the antibody-copolymer conjugate on a molar basis.

In this step, the specimen contained in the mixture solution is not particularly limited and may be provided from a biological sample. As one embodiment, a biological sample including the specimen may be added directly to the mixture solution.

In general, biological samples contain many impurities. In common nucleic acid extraction, treatment with a proteolytic enzyme or the like is often performed in order to inactivate or digest these impurities.

However, in the present nucleic acid extraction method, even in the absence of a proteolytic enzyme, the specimen is captured by the antibody-copolymer conjugate and can be easily separated from impurities in step S2 described later, so nucleic acid extraction can be performed with sufficient efficiency even without performing treatment with a proteolytic enzyme or the like.

### <Step S2>

Returning to the flowchart in Fig. 1, next, as step S2, the mixture solution containing the specimen and the antibody-copolymer conjugate obtained in step S1 is heated to cause aggregation of the antibody-copolymer conjugate that captures the specimen, and a nucleic acid encapsulated in the specimen is extracted. In this case, a carrier nucleic acid is not necessarily added but may be added.

The heating temperature may be adjusted as appropriate depending on the LCST of the specific copolymer and is preferably 20 to 40°C.

Common procedures for extracting a nucleic acid encapsulated in a specimen include lysis of tissue with a proteolytic enzyme and inactivation of undesired enzymes, washing, filtering, fractionation of components, and the like. The manipulation needs to be performed over multiple stages. In nucleic acid extraction for PCR testing of SARS-CoV-2, this process can generally take about 90 minutes. In addition, the use of various reagents tends to increase the cost required for a single extraction operation.

On the other hand, the inventors of the present invention have found that surprisingly, the present nucleic acid extraction method can extract a nucleic acid encapsulated in a specimen even in the absence of a proteolytic enzyme and even without using a carrier nucleic acid. The mechanism for this is not necessarily clear, but presumably, the specimen is captured through the interaction with the antibody of the antibody-specific copolymer conjugate and allowed to aggregate, whereby the separation of impurities and the concentration of the specimen can be performed simultaneously.

The present nucleic acid extraction method can extract a nucleic acid encapsulated in a specimen with a simple manipulation.

Specifically, the removal of impurities and the concentration of the specimen are carried out together by allowing the antibody-specific copolymer conjugate that captures the specimen to aggregate by heating, and obtaining the solid content by centrifugation or the like. Then, a nucleic acid encapsulated in the specimen may be released by a known method for the obtained solid content. Examples of such a method include, but not limited to, a method using a nucleic acid-binding solid carrier such as silica particles and a chaotropic agent (J. Clin. Microbiol., vol. 28 No. 3, p. 495-503 (1990)).

More specifically, nucleic acids are adsorbed on a nucleic acid-binding solid carrier in the presence of a chaotropic agent, the carrier is then washed with a washing solution, and the nucleic acids are eluted from the carrier using water or a low-salt buffer. These methods are well known to those skilled in the art, and commercially available kits may be used.

The nucleic acid extracted by the present nucleic acid extraction method can be subjected to nucleic acid amplification.

Examples of the method for amplifying DNA extracted from specimens include methods that involve a heat denaturation process of double-stranded DNA such as a polymerase chain reaction (PCR) and a ligase chain reaction (LCR); and isothermal amplification methods that do not involve a heat denaturation process of double-stranded DNA such as loop-mediated isothermal amplification (LAMP), strand displacement amplification (SDA), isothermal and chimeric primer-initiated amplification of nucleic acids (ICAN), a smart amplification process (SMAP), and self-sustained sequence replication (3SR).

Examples of the method for amplifying RNA extracted from specimens include transcription mediated amplification (TMA), nucleic acid sequence-based amplification (NASBA), and a transcription-reverse transcription concerted reaction (TRCR).

### [Nucleic acid amplification method]

An embodiment of the nucleic acid amplification method includes extracting the nucleic acid from the specimen using the above nucleic acid extraction method and amplifying the extracted nucleic acid using a polymerase chain reaction (PCR). The method of amplifying DNA by PCR is known and can be performed, for example, with reference to Science 239, 487-491 (1988).

When the specimen is an RNA virus or the like, a reverse transcription reaction can be performed to synthesize DNA complementary to the extracted RNA before amplifying DNA using PCR. The method of reverse transcription reaction is known, and the reaction temperature is the temperature at which a thermostable reverse transcriptase is active, and is preferably 35 to 90°C as one embodiment. The reverse transcription reaction time can be set as appropriate considering, for example, the strand length of the cDNA to be synthesized.

The extraction of RNA from the specimen, the reverse transcription reaction, and the amplification reaction of the target nucleic acid may be performed sequentially or consecutively (in one step). When the reverse transcription reaction and the amplification reaction of the target nucleic acid are performed consecutively, it is preferable to use a thermostable reverse transcriptase and a thermostable DNA polymerase. Various polymerases that can be used for PCR can be used as the thermostable DNA polymerase. A thermostable DNA polymerase with thermostable reverse transcriptase activity can also be used.

A primer pair can also be used to amplify the target region of the cDNA. In this case, one of the primers may be shared with the primer for a reverse transcription reaction in the reverse transcription reaction. Furthermore, when there are multiple regions of the target nucleic acid, multiple primer pairs therefor may be used in combination.

The present nucleic acid amplification method can extract a nucleic acid with a simple manipulation without using a proteolytic enzyme, thereby eliminating a bottleneck and enabling rapid nucleic acid amplification.

### [Nucleic acid extraction kit]

An embodiment of the nucleic acid extraction kit is a nucleic acid extraction kit to be used for extracting a nucleic acid from a specimen in the absence of a proteolytic enzyme and includes a first agent including a specific copolymer and a second agent including an antibody-linker complex.

The first agent includes, for example, a container and the specific polymer contained in the container and may be solid (e.g., powdery) or liquid including the specific polymer. When the first agent is liquid, an exemplary form thereof includes a specific polymer and water (an aqueous solution containing a buffering agent).

The second agent includes, for example, a container and the antibody-linker complex contained in the container. The second agent may be liquid and an exemplary form thereof includes the antibody-linker complex and a buffer solution (e.g., phosphate buffered saline, PBS). The content of the antibody-linker complex in the second agent is not particularly limited and is preferably 0.01 to 10000 µg/mL.

An antibody-copolymer conjugate can be generated by mixing the first agent and the second agent. The mixing ratio between the first agent and the second agent is not particularly limited and is adjusted so that the ratio (antibody/specific copolymer) of the content on a molar basis of the specific copolymer included in the first agent to the content on a molar basis (amount of substance basis) of the antibody (bound to the linker) included in the second agent is preferably adjusted to be 1 to 200 and more preferably adjusted to be 10 to 50.

The amount of each agent may be adjusted so that the antibody/specific copolymer is within the above numerical range when all of the first agent and all of the second agent included in the present nucleic acid extraction kit are mixed.

With the present nucleic acid extraction kit, a nucleic acid included in a specimen can be extracted and concentrated by preparing a mixture solution containing the first agent, the second agent, and the specimen to generate an antibody-copolymer conjugate, and then heating the mixture solution. For example, when the specimen is an RNA virus, the first agent and the second agent are added to saliva or the like containing an RNA virus and allowed to react, and then upon heating, the RNA virus captured by the antibody-copolymer conjugate aggregates and precipitates together with the antibody-copolymer conjugate. By solid-liquid separation, impurities (liquid phase) are removed and the specimen is concentrated. RNA is then extracted from the concentrated specimen.

With the present nucleic acid extraction kit, a nucleic acid can be extracted in the absence of a proteolytic enzyme and with a simple manipulation. Even from a biological sample or the like with a low content of a specimen (e.g., RNA virus), a nucleic acid can be extracted efficiently by the specimen capture and aggregation function of the antibody-copolymer conjugate.

### [PCR test kit]

An embodiment of the PCR test kit may include any other components as long as it includes the above nucleic acid extraction kit. For example, when the specimen is an RNA virus, the other components may include, for example, a reverse transcriptase for cDNA synthesis, a probe for real-time detection, an intercalator, a buffer, deoxyribonucleotides such as dNTP, a surfactant, salts, and a primer.

A plurality of these components may be mixed and provided as a premix.

With the present PCR test kit, a nucleic acid can be easily extracted from a specimen included in a biological sample or the like in the absence of a proteolytic enzyme and can be amplified. The use of the present PCR test kit considerably can reduce the time required for a PCR test and the necessary costs.

### [Examples]

The embodiments will be described in more details below with non-limiting examples.

### [Synthesis example of specific copolymer]

### - Synthesis of HIPAAm

For synthesis of the specific copolymer, "HIPAAm" was synthesized by the following procedure as a preparation. "HIPAAm" is an abbreviation derived from "hydroxy isopropyl acrylamide".

D,L-2-amino-1-propanol (0.15 mol) and triethylamine (0.15 mol) were dissolved well in anhydrous chloroform and stirred at 5°C for 20 minutes, and acryloyl chloride (0.15 mol) was then slowly added, followed by stirring at 5°C for 2 hours.

After evaporation of the solvent, the resulting product was redissolved in 2-propanol, and the solution was kept at -20°C for 24 hours or longer. Finally, salts were removed by filtration, followed by concentration and purification by column chromatography. Synthesis of HIPAAm was verified by thin layer chromatography (TLC) and ¹H NMR (nuclear magnetic resonance) (solvent was D₂O).

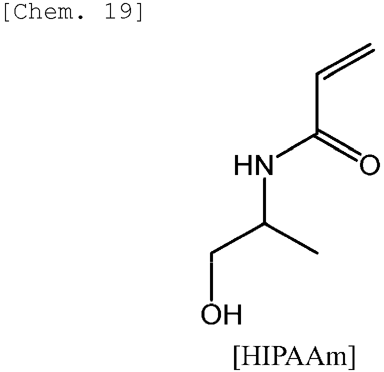

### - Synthesis of P(NIPAAm-co-HIPAAm)

"P(NIPAAm-co-HIPAAm)" was synthesized by the following procedure as a polymer that serves as a precursor compound for the specific copolymer.

P(NIPAAm-co-HIPAAm) was synthesized by RAFT polymerization of HIPAAm and NIPAAm (see the following scheme for structure) as shown by the following scheme. A solution containing 1.89 g of NIPAAm, 0.11 g of HIPAAm, 1.31 mg of AIBN, 12.7 mg of cyanomethyl dodecyl trithiocarbonate (CDT), and 17.6 ml of ethanol was stirred at 20°C for 20 hours, followed by evaporation and vacuum drying.

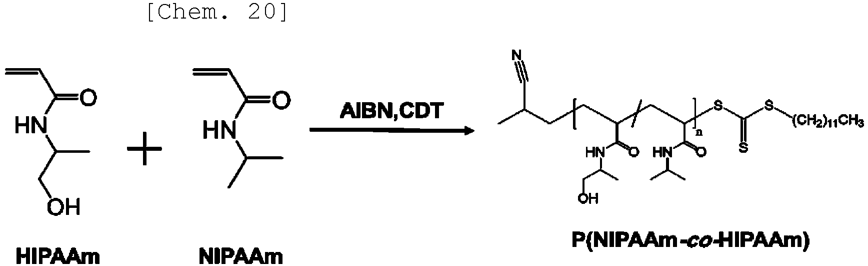

### - Synthesis of P(NIPAAm-co-HIPAAm-co-SAKIPAAm)

P(NIPAAm-co-HIPAAm-co-SAKIPAAm) was synthesized as the specific polymer.

As shown by the following scheme, dibenzylcyclooctanoic acid (DBCO-acid) having a click reaction site was introduced into the hydroxy group of HIPAAm by dehydration condensation to obtain a clickable responsive polymer P(NIPAAm-co-HIPAAm-co-SAKIPAAm).

A solution containing 30 ml of dichloromethane (DCM), 35.8 mg of DBCO acid, 12 mg of 4-dimethylaminopyridine (DMAP), 100 mg of P(NIPAAm-co-HIPAAm), and 20 mg of N',N'-dicyclohexylcarbodiimide (DCC) was stirred overnight, followed by evaporation and vacuum drying.

The obtained P(NIPAAm-co-HIPAAm-co-SAKIPAAm) had a number average molecular weight of 2.01 × 10⁴ and an LCST of about 30°C.

The content of repeating units determined by ¹H-NMR was NIPAAm:HIPAAm:SAKIPAAm (on a molar basis) = 96.4:1.2:2.4. The notation "NIPAAm" in P(NIPAAm-co-HIPAAm-co-SAKIPAAm) means a unit based on NIPAAm and corresponds to the unit represented by formula 1. The same applies to the notation "HIPAAm", which corresponds to the unit represented by formula 6. The same applies to the notation "SAKIPAAm", which corresponds to the unit represented by formula 2.

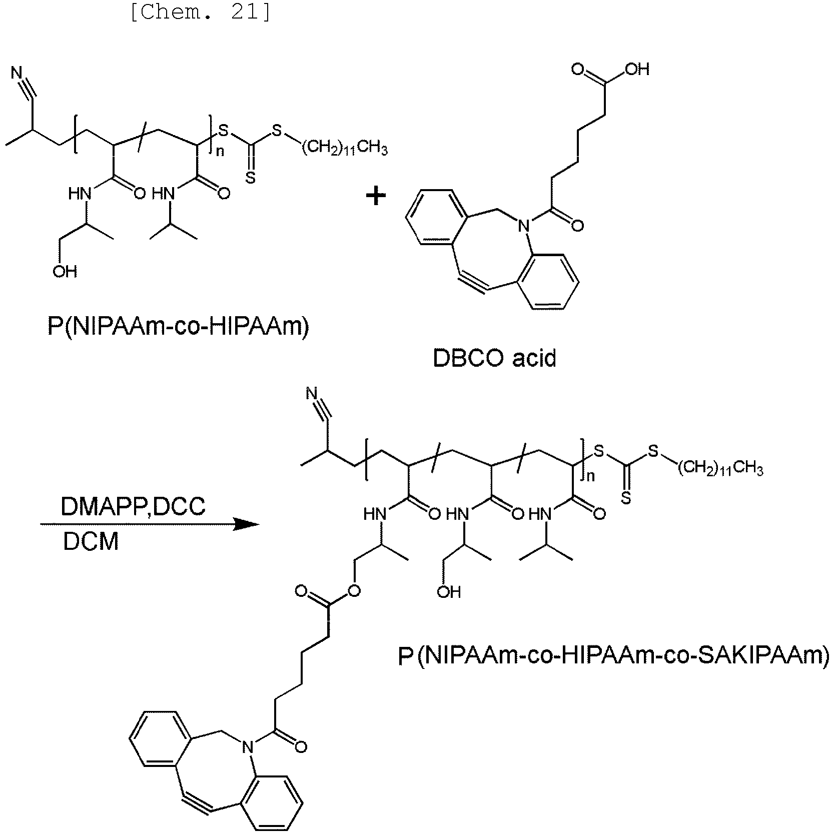

### [Synthesis of antibody-linker complex]

Azido-PEG4-NHS ester (azido-ethylene glycol (EG4)-NHS ester, Tokyo Chemical Industry Co., Ltd.) powder was dissolved in DMSO (10 mg/ml, dimethyl sulfoxide).

Instead of an anti-COVID-19 monoclonal antibody (>95%, manufactured by MyBioSource, anti-COVID-19 antibody: antiviral COVID 19 nucleocapsid (NP) humanized coronavirus monoclonal antibody), "SARS-CoV-2 spike protein (S1-NTD) antibody #56996" (Cell Signaling Technology) was dispersed in carbonate buffer (PH 8.6) and stirred at 4°C for 5 hours to synthesize an antibody-linker complex.

The antibody/linker ratio was set to 1:100.

### [Collection of biological sample]

Biological samples were collected from patients (325) recruited between October 2021 and April 2022 at the outpatient department of the Egyptian Liver Research Institute and Hospital. These patients included 92 COVID-19 patients (50 males and 42 females). Nasopharyngeal swabs were collected from all the participants and stored in a prescribed transport medium for SARS-CoV-2 specimen transport at -80°C until testing.

The entry criteria for the testing were as follows.
- Age 18 or over
- Willingness and ability to provide written informed consent.
- Person who is positive, negative, or suspected positive for COVID-19 by PCR or antigen test.

The exclusion criteria were as follows.
- Age under 18
- Person who does not agree to give informed consent

The above research protocol was approved by the research ethics committee of the Egyptian Liver Research Institute and Hospital, and the protocol and conduct of the research were compliant with "CIOMS/WHO. International Ethical Guidelines for Biomedical Research Involving Human Subjects. Geneva: CIOMS. 1993." and its revised version and with written informed consent from all the patients.

### [Example 1]

The synthesized specific copolymer and antibody-linker complex were used to extract a virus RNA from a biological sample in the absence of a proteolytic enzyme. The procedure is as follows.

### (Step 1)

First, 900 µL of a sample was mixed with the antibody-linker complex (10 µg/mL, 100 µL PBS solution) and incubated at 37°C for 1 hour.

Next, P(NIPAAm-co-HIPAAm-co-SAKIPAAm) (antibody:specific copolymer ratio = 1:30, Mn = 2.01 × 10⁴ g/mol) was conjugated to the antibody-linker complex through a click reaction at 4°C for 1 hour.

The mixture solution was then transferred to a 2.0 mL microtube and centrifuged at 37°C at 13,800 ×g for 5 minutes. The concentrated precipitate was collected and the supernatant was discarded. Next, the concentrated precipitate and 200 µL of PBS were added to a lysis tube (LT).

### (Step 2)

To this, 250 µL of ethanol (96 to 100%) was further added.

The mixture was stirred (vortex) for approximately 15 seconds and then incubated at room temperature (15 to 25°C) for 5 minutes. Subsequently, the droplets were removed by centrifugation.

The lysate was then transferred to a "QIAamp MinElute column" and centrifuged at 6000 ×g for 1 minute or longer.

Then, the wash tube containing the filtrate was discarded, 500 µL of "Buffer AW1 (QIAGEN)" was added, and centrifugation was performed at 6000 ×g for 1 minute or longer. Then, the wash tube containing the filtrate was discarded, 500 µL of "Buffer AW2 (QIAGEN)" was added, and centrifugation was performed at 6000 ×g for 1 minute or longer.

Then, the wash tube containing the filtrate was discarded, 500 µL of ethanol (96 to 100%) was added, and centrifugation was performed at 6000 ×g for 1 minute or longer. Then, the wash tube containing the filtrate was discarded, and the QIAamp MinElute column was transferred to a clean 2 ml wash tube (WT).

Then, centrifugation was performed at about 20,000 ×g for 3 minutes to dry the membrane. Then, the "QIAamp MinElute column" was transferred to a new 2 ml wash tube (WT). Then, with the lid open, incubation was performed at 56°C for 3 minutes to allow the membrane to dry completely. The "QIAamp MinElute column" was then transferred to an elution tube (ET) and the filtrate was discarded. Then, 20 to 150 µL of "Buffer AVE (QIAGEN)" was added to the center of the membrane, which was incubated at room temperature for 5 minutes. Then, centrifugation was performed at about 20,000 ×g for 1 minute or longer.

The RNA extracted by the above procedure was subjected to RT-PCR (PCR with reverse transcription) test. Specifically, cDNA was synthesized using "SARS-CoV-2/SARS-CoV Multiplex (manufactured by DNA-Technology)" and quantified using "DTlite real-time PCR (manufactured by DNA-Technology)". The thermal cycler program and the like followed the manuals of the above kit.

### [Comparative Example 1]

A virus RNA was extracted from the same biological sample as in Example 1 using a proteolytic enzyme without using the specific copolymer or the antibody-linker complex. The detailed procedure is the same as the procedure in Example 1, except that (step 1) is as follows.

First, 25 µL of protease was added to a lysis tube (LT). Then, 200 µL of a sample was added to the lysis tube. Further, 200 µL of "Buffer AL (QIAGEN)" containing 28 µg/mL of a carrier RNA was added and stirred for approximately 15 seconds. Then, the mixture was incubated in a heating block at 56°C for 15 minutes, and then centrifuged to remove droplets. The subsequent step (step 2) was the same as in Example 1.

### [Reference Example 1]

A virus RNA was extracted from the same biological sample as in Example 1 using the specific copolymer and the antibody-linker complex and further using a proteolytic enzyme. The detailed procedure is the same except that (step 1) in Example 1 was modified as follows.

First, 900 µL of a sample was mixed with the antibody-linker complex (10 µg/mL, 100 µL PBS solution) and incubated at 37°C for 1 hour.

Next, P(NIPAAm-co-HIPAAm-co-SAKIPAAm) (antibody:specific copolymer ratio = 1:30, Mn = 2.01 × 10⁴ g/mol) was conjugated to the antibody-linker complex through a click reaction at 4°C for 1 hour.

The mixture was then transferred to a 2.0 mL microtube and centrifuged at 37°C at 13,800 ×g for 5 minutes. The concentrated precipitate was collected and the supernatant was discarded.

Next, 25 µL of protease was added to a lysis tube (LT). To this, 200 µL of the sample with the concentrated precipitate was added. Further, 200 µL of "Buffer AL (QIAGEN)" containing 28 µg/mL of a carrier RNA was added and stirred for approximately 15 seconds. Then, the mixture was incubated in a heating block at 56°C for 15 minutes, and then centrifuged to remove droplets. The subsequent step (step 2) was the same as in Example 1.

### [Results]

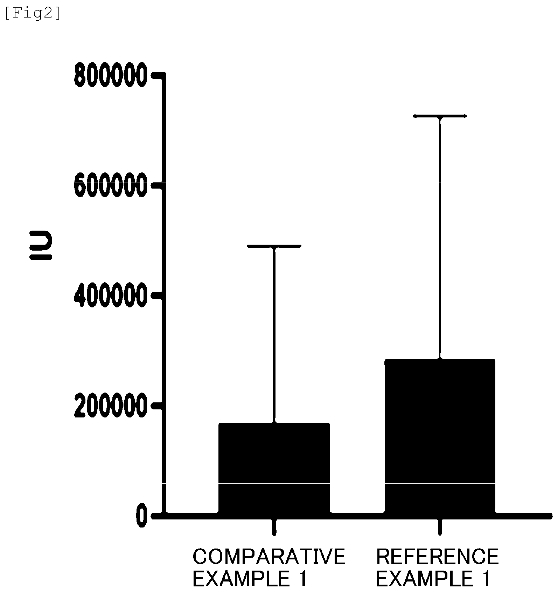

Fig. 2 illustrates the quantification result by a real-time PCR method ("SLAN-96P real-time PCR system") using the nucleic acids extracted by the method of Comparative Example 1 and the method of Reference Example 1.

The result in Fig. 2 showed that the quantification result obtained in Reference Example 1 using the specific copolymer and the antibody-linker complex was about 1.5 times greater than the quantification result obtained in Comparative Example 1, indicating that the specimen was concentrated with the specific copolymer and the antibody-linker complex.

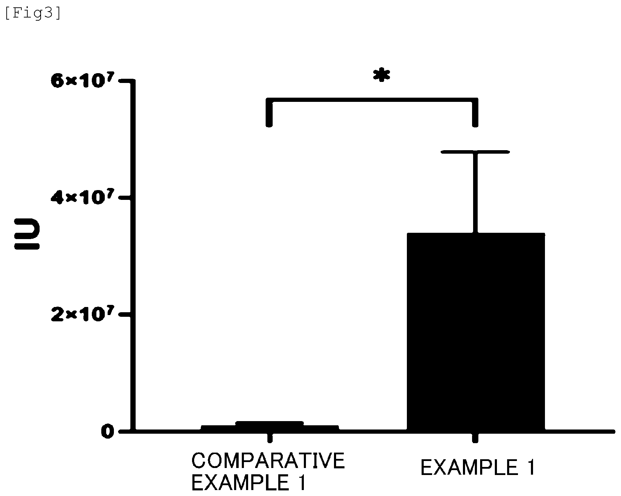

Fig. 3 illustrates the quantification result by a real-time PCR method using the nucleic acids extracted by the method of Comparative Example 1 and the method of Example 1. The quantification result obtained in Example 1 was about 30 times greater than the quantification result obtained in Comparative Example 1. The result in Fig. 3 showed that the target nucleic acid can be detected with sufficient sensitivity even without using a proteolytic enzyme or a carrier nucleic acid.

## Claims

1. A nucleic acid extraction method comprising,
in absence of a proteolytic enzyme:
preparing a mixture solution including
at least one specimen selected from the group consisting of cells, extracellular vesicles, and virions,
a copolymer including a repeating unit represented by formula 1 below and a repeating unit represented by formula 2 below, and
an antibody-linker complex obtained by binding an antibody that binds to the specimen and a linker represented by formula 3 below via an amide bond, and
generating an antibody-copolymer conjugate; and
heating the mixture solution to allow the antibody-copolymer conjugate to aggregate, and extracting a nucleic acid encapsulated in the specimen: 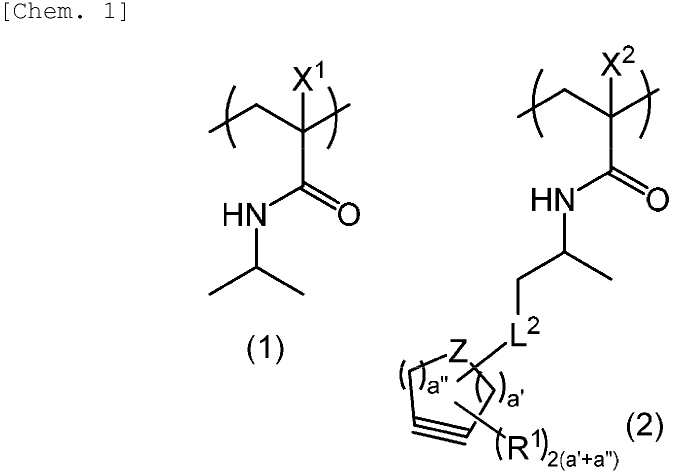 (in formula 1, X¹ represents a hydrogen atom or a linear or branched alkyl group having 1 to 6 carbon atoms; in formula 2, X² represents a hydrogen atom or a linear or branched alkyl group having 1 to 6 carbon atoms, L² represents a divalent group, R¹ is selected from the group consisting of a hydrogen atom, a halogen atom, -OR⁵, -NO₂, -CN, -S(O)₂R⁵, an alkyl group having 1 to 24 carbon atoms, an alkenyl group having 2 to 24 carbon atoms, and a (hetero)aryl group having 6 to 24 carbon atoms, a plurality of R¹s are same or different from each other, two or more of R¹s are optionally bonded to each other to form a ring, where R⁵ is selected from the group consisting of a hydrogen atom, a halogen atom, an alkyl group having 1 to 24 carbon atoms, and a (hetero)aryl group having 6 to 24 carbon atoms, Z is selected from the group consisting of C(R¹)₂, O, S, and NR¹, a' is an integer of 0 to 8, a" is an integer of 0 to 8, and a sum of a' and a" is less than 10) 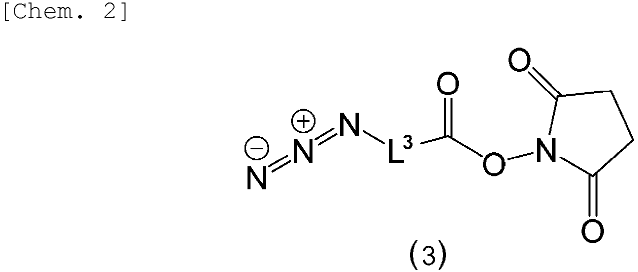 (in formula 3, L³ represents a divalent hydrocarbon group optionally having a heteroatom).

2. The nucleic acid extraction method according to claim 1, wherein a content of the repeating unit represented by the formula 2 is 1.0 to 30.0 mol% when a total of the repeating units of the copolymer is defined as 100 mol%.

3. The nucleic acid extraction method according to claim 1 or 2, wherein the repeating unit represented by the formula 2 is at least one repeating unit selected from the group consisting of a repeating unit represented by formula 4 below and a repeating unit represented by formula 5 below: 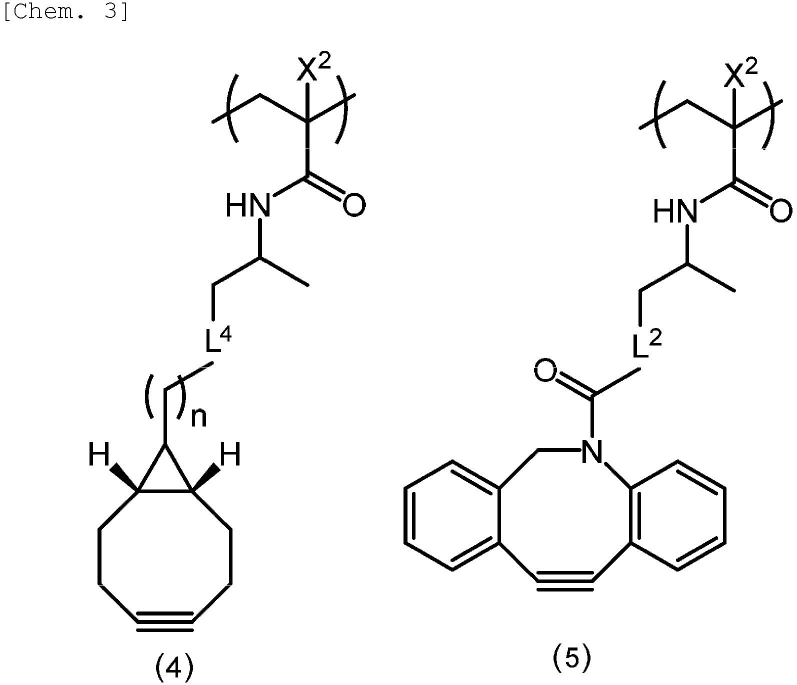 (in formula 4 and formula 5, X² represents a hydrogen atom or a linear or branched alkyl group having 1 to 6 carbon atoms; in formula 4, L⁴ is one group selected from the group consisting of -O-, -S-, and -NR²-, where R² represents a hydrogen atom or an alkyl group having 1 to 6 carbon atoms, n represents an integer of 1 to 10; and in formula 5, L² represents a divalent group).

4. The nucleic acid extraction method according to any one of claims 1 to 3, wherein the copolymer further includes a repeating unit represented by formula 6 below: 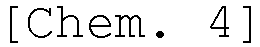 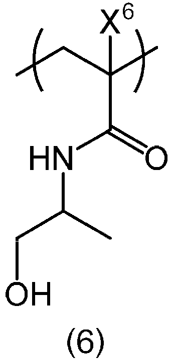 (in formula 6, X⁶ represents a hydrogen atom or a linear or branched alkyl group having 1 to 6 carbon atoms).

5. The nucleic acid extraction method according to any one of claims 1 to 4, wherein a content of the repeating unit represented by the formula 2 is 2.0 to 30.0 mol% when a total of repeating units of the copolymer is defined as 100 mol%.

6. The nucleic acid extraction method according to any one of claims 1 to 5, wherein the copolymer has a number average molecular weight of 5000 to 50000.

7. The nucleic acid extraction method according to any one of claims 1 to 6, wherein a ratio of a content of the copolymer to a content of the antibody contained in the antibody-linker complex in the mixture solution is 0.5 to 30.0 on a molar basis.

8. The nucleic acid extraction method according to any one of claims 1 to 7, wherein the antibody-copolymer conjugate includes a repeating unit represented by formula 1 below and a repeating unit represented by formula 7 below: 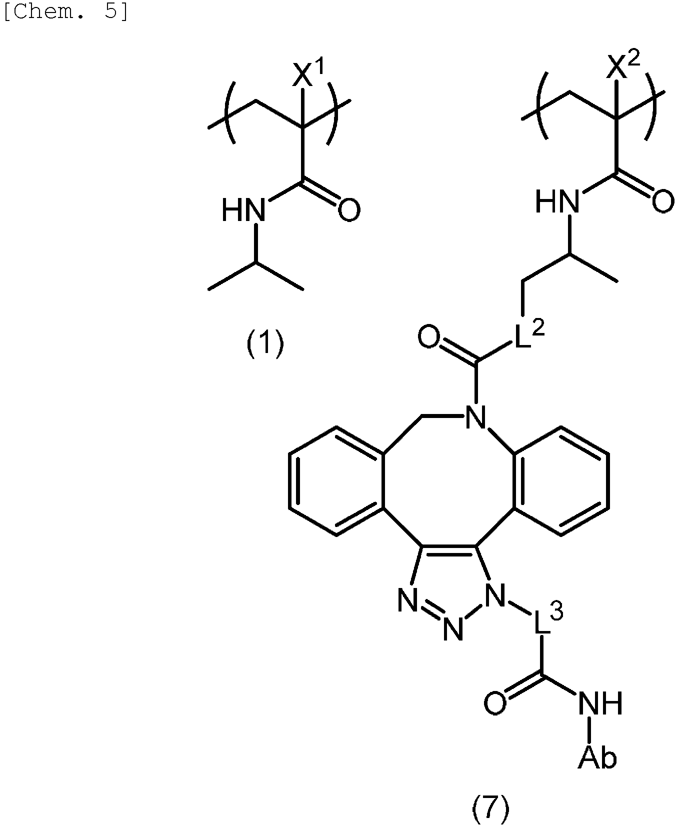 (in formula 1, X¹ represents a hydrogen atom or a linear or branched alkyl group having 1 to 6 carbon atoms; in formula 7, X² represents a hydrogen atom or a linear or branched alkyl group having 1 to 6 carbon atoms, L² represents a divalent group, L³ represents a divalent hydrocarbon group optionally having a heteroatom, and Ab represents a residue of the antibody).

9. The nucleic acid extraction method according to any one of claims 1 to 8, wherein the heating is heating to increase a temperature of the mixture solution to 20 to 40°C.

10. The nucleic acid extraction method according to any one of claims 1 to 9, wherein the specimen is a membrane structure having a lipid bilayer.

11. The nucleic acid extraction method according to any one of claims 1 to 9, wherein the specimen is an enveloped virus.

12. The nucleic acid extraction method according to any one of claims 1 to 9, wherein the specimen is SARS-CoV-2 virus.

13. A nucleic acid amplification method comprising:
extracting the nucleic acid from the specimen using the nucleic acid extraction method according to any one of claims 1 to 12; and
amplifying the extracted nucleic acid through a polymerase chain reaction.

14. A nucleic acid extraction kit to be used for extracting a nucleic acid from at least one specimen selected from the group consisting of cells, extracellular vesicles, and virions, in absence of a proteolytic enzyme, the nucleic acid extraction kit comprising:
a first agent including a copolymer including a repeating unit represented by formula 1 below and a repeating unit represented by formula 2 below; and
a second agent including an antibody-linker complex obtained by binding an antibody that binds to the specimen and a linker represented by formula 3 below via an amide bond: 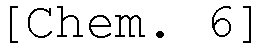 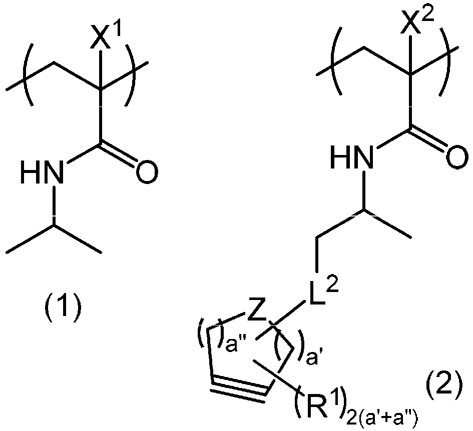 (in formula 1, X¹ represents a hydrogen atom or a linear or branched alkyl group having 1 to 6 carbon atoms; in formula 2, X² represents a hydrogen atom or a linear or branched alkyl group having 1 to 6 carbon atoms, L² represents a divalent group, R¹ is selected from the group consisting of a hydrogen atom, a halogen atom, -OR⁵, -NO₂, -CN, -S(O)₂R⁵, an alkyl group having 1 to 24 carbon atoms, an alkenyl group having 2 to 24 carbon atoms, and a (hetero)aryl group having 6 to 24 carbon atoms, a plurality of R¹s are same or different from each other, two or more of R¹s are optionally bonded to each other to form a ring, where R⁵ is selected from the group consisting of a hydrogen atom, a halogen atom, an alkyl group having 1 to 24 carbon atoms, and a (hetero)aryl group having 6 to 24 carbon atoms, Z is selected from the group consisting of C(R¹)₂, O, S, and NR¹, a' is an integer of 0 to 8, a" is an integer of 0 to 8, and a sum of a' and a" is less than 10) 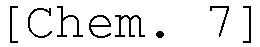 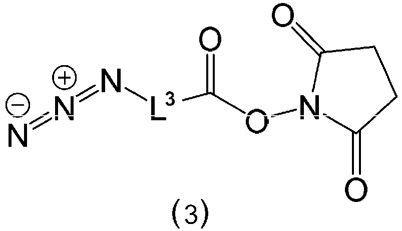 (in formula 3, L³ represents a divalent hydrocarbon group optionally having a heteroatom).

15. A PCR test kit comprising the nucleic acid extraction kit according to claim 14.
